# EUROPÄISCHE PATENTSCHRIFT

(11) **EP 3 872 162 B1**
(45) Veröffentlichungstag und Bekanntmachung des Hinweises auf die Patenterteilung: **06.11.2024**
(21) Anmeldenummer: 20159456.1
(22) Anmeldetag: 26.02.2020
(51) Int. Cl.: C12M 1/21, H03K 17/96

(54) **ERFASSUNG VON SCHAUM IN EINER BIOREAKTORANLAGE**
DETECTION OF FOAM IN A BIOREACTOR SYSTEM
DÉTECTION DE LA MOUSSE DANS UNE INSTALLATION DE BIORÉACTEUR

(43) Veröffentlichungstag der Anmeldung: 01.09.2021
(73) Patentinhaber: Sartorius Stedim Biotech GmbH, 37079 Göttingen (DE)
(72) Erfinder: Prediger, Andreas, 37073 Göttingen (DE); Götemann, Sina, 37120 Bovenden (DE); Leupold, Marco, 37075 Göttingen (DE); Scholz, Jochen, 37077 Göttingen (DE)
(74) Vertreter: Novagraaf International SA

(56) Entgegenhaltungen:
- DE-A1- 102015 114 510
- DE-U1- 202011 107 423
- US-A- 3 306 341
- US-A- 3 427 252
- US-A1- 2017 184 421

## Beschreibung

Die Erfindung betrifft ein System, ein Verfahren und eine kapazitive Sensoreinheit zum Erfassen des Vorhandenseins von Schaum eines Mediums an zumindest einer Position in einer Bioreaktoranlage und ein Verfahren zur Montage eines Systems zum Erfassen eines Vorhandenseins von Schaums in einer Bioreaktoranlage.

Die Überwachung von Bioreaktoren und Bioreaktoranlagen hinsichtlich der Erzeugung von Schaum ist für den Ablauf eines Prozesses innerhalb des Bioreaktors wichtig, da ggf. ein Zerplatzen von Schaumblasen Scherkräfte verursacht, die Zellen und Proteine schädigen können. Ein solcher Prozess kann insbesondere einen biochemischen Prozess, beispielsweise einen Fermentationsprozesses aufweisen. Ebenfalls kann es vorkommen, dass ein Material bzw. eine Substanz das in einem Schaum vorliegt im Wesentlichen nicht den vorbestimmten Prozessschritten unterliegt. So kann ein Schaum ggf. weniger gut durchmischt werden als ein fluides Medium, insbesondere eine Flüssigkeit. Auch eine Temperatur kann innerhalb eines Schaums weniger gut kontrolliert werden als in einer Flüssigkeit. Darüber hinaus besteht das Risiko, dass nicht erkannter Schaum beispielsweise in den Abluftfilter einer Bioreaktoranlage wächst und dort einen Filter blockiert. Dies kann ggf. zu einem sehr schnellen Druckanstieg in der Bioreaktoranlage führen. Eine weitere Gaszufur ist dann nicht mehr möglich und somit wird eine Regelung des Sauerstoffgehalts des flüssigen Mediums verhindert. Als Resultat dessen kann es vorkommen, dass die gesamte Medienfüllung der Bioreaktoranlage verworfen werden muss. Dies kann im schlimmsten Fall zu Produktionsausfällen und hohen Kosten führen. Deshalb stellt Schaum in Bioprozessen ein Problem dar.

Schaum wird als Störgröße beispielsweise in der Pharmaindustrie mittels Leitfähigkeitssensoren erfasst. Diese haben jedoch den Nachteil, dass sie in direktem Kontakt mit dem Schaum kommen müssen um diesen erfassen zu können.

Abhängig von Prozessbedingungen und eingesetzten Organismen können ggf. Schäume mit unterschiedlichen Eigenschaften entstehen und insbesondere unterschiedlich schnell wachsen. Aus diesem Grund werden Schaumsensoren und Antischaum-Mittel regelmäßig eingesetzt. Es werden gegenwärtig schon Biorektoren hinsichtlich des Vorliegens von Schaum überwacht. So beschreibt z.B. die Patentschrift DE 102010007559 A1 einen Bioreaktorbehälter mit einem optischen Schaumsensor, der eine Schaumanlagefläche zur Anlage des zu detektierenden Schaums aufweist. Auch aus der EP 1 950 281 B1 sind Bioreaktorbehälter mit optischen Schaumsensoren bekannt.

DE 20 2011 107423 U1 offenbart eine Vorrichtung zur Detektion von Schaum in einem aus einem elektrisch isolierenden Material gefertigten Behälter, mit einer Sensorelektrode, mit mindestens einer Schutzelektrode, und mit einer Elektronikeinheit, welche zumindest die Sensorelektrode mit einem Steuersignal beaufschlagt und welche von der Sensorelektrode ein Antwortsignal empfängt und auswertet, wobei die Sensorelektrode auf eine Trägerfolie aufgebracht ist, wobei die Sensorelektrode von einer isolierenden Schicht bedeckt ist, wobei die Schutzelektrode derart auf der isolierenden Schicht aufgebracht ist, dass sie die Sensorelektrode zumindest teilweise bedeckt, und wobei die Trägerfolie auf eine Außenwandung des Behälters aufbringbar ist. US 3 306 341 A offenbart eine Säule mit Vorrichtung zur Detektion von Schaum. US 2017/184421 A1 offenbart ein kapazitives Sensorelement, welches eine komprimierbare Isolatorschicht aufweist, die eine erste Leiterspuranordnung auf einer ersten Oberfläche und eine zweite Leiterspuranordnung auf einer zweiten Oberfläche aufweist. Die zweite Fläche liegt gegenüber der ersten Fläche. Die Sensorvorrichtung umfasst ferner eine Auswertungsvorrichtung zum wiederholten Erfassen eines Kapazitätswertes des Sensorelements. DE 10 2015 114510 A1 beschreibt eine Vorrichtung zum Verringern der Schaumbildung und/oder Verbesserung der Entgasung vom Gärmedium in einem Biogasfermenter mittels einer Vibrationsvorrichtung. US 3 427 252 A beschreibt ein Antischaum-Verteilungssystem umfassend eine Mehrzahl von Ventilen, durch welche Antischaum-Mittel fließt, wobei die einzelnen Ventile durch in graduellen Abständen zu der Oberfläche der Prozessflüßigkeit angeordneten Sensoren gesteuert werden.

Es besteht jedoch noch ein Bedarf insbesondere schnellwachsende Schäume innerhalb von Bioreaktoranlagen zuverlässig und effizient erfassen zu können. Deshalb besteht die Aufgabe gemäß eines Aspekts darin, ein zuverlässiges und effizientes System zum Erfassen von Schaum bereitzustellen. Analog besteht die Aufgabe gemäß anderer Aspekte auch darin, ein Verfahren und eine kapazitive Sensoreinheit zum Erfassen von Schaum und ein Verfahren zur Montage eines Systems zum Erfassen von Schaum mit jeweils verbesserter Zuverlässigkeit und Effizienz bereit zu stellen. Insbesondere soll Schaumwachstum in Bioreaktoranlagen mit Einweg-Behältern erfasst werden, da immer öfter Einweg-Behälter in Bioreaktoranlagen zum Einsatz kommen. Insbesondere soll der Schaum erfasst werden können ohne dass der Sensor in direkten Kontakt mit dem Schaum bzw. der Kultivierungsflüssigkeit gebracht werden muss. Die Messung soll also möglichst nichtinvasiv sein.

Gemäß einem Aspekt umfasst ein System zum Erfassen zumindest eines Schaums bzw. des Vorhandenseins von Schaum eines Mediums, beispielsweise eines Schaumniveaus in einer Bioreaktoranlage:
- eine Bioreaktoranlage mit zumindest einem Einwegbehälter, insbesondere einem Einwegbeutel zum Aufnehmen des Mediums, das den Schaum aufweisen kann und insbesondere den Schaum erzeugen kann;und
- zumindest zwei kapazitive Sensoreinheiten, die an zumindest zwei unterschiedlichen Anordnungspositionen bzw. Messpositionen der Bioreaktoranlage angebracht sind und/oder angebracht werden können,
wobei die kapazitiven Sensor-Einheiten jeweils zumindest ein Elektrodensystem für eine kapazitive Messung, insbesondere für eine Permittivitätsmessung aufweisen und das Vorhandensein von Schaum an den zumindest zwei Anordnungspositionen auf Basis der kapazitiven Messung erfassen können; und
wobei die kapazitive Sensor-Einheiten dazu ausgelegt sind, erfasste Daten betreffend das Vorhandensein von Schaum an zumindest eine Kontrolleinheit zum Überwachen, zum Steuern und/oder Regeln einer Schaumbildung zu übermitteln, wobei die Kontrolleinheit insbesondere mit einer Recheneinheit und/oder einer Steuer- und/oder Regeleinheit zum Kontrollieren oder Erfassen, jeweils insbesondere zum Überwachen, zum Steuern und/oder Regeln einer Schaumbildung in der Bioreaktoranlage auf der Basis der erfassten Daten ausgestattet ist.

Bevorzugt kann durch die Anbringung der kapazitiven Sensoreinheiten an spezifischen Punkten auf das Schaumniveau rückgeschlossen werden, da der Schaum im Bioreaktor häufig gleichmäßig ansteigt. Durch die Anbringung an mehreren Punkten kann das Schaumniveau stufenweise überwacht werden.

Das System hat den Vorteil, dass die Schaumproduktion an mehreren Anordnungspositionen ermittelt und ggf. dauerhaft überwacht werden kann, sodass beispielsweise lokal an der Stelle des Vorhandenseins eines Schaums bzw. an der Stelle der Schaumproduktion Gegenmaßnahmen zur Reduktion von Schaum getroffen werden können. So kann z.B. eine Substanz zum Verhindern oder zumindest zum Vermindern der Schaumproduktion bzw. ein Antischaum-Mittel an der entsprechenden Stelle in der Bioreaktoranlage eingebracht und/ oder in kritischen Situationen und Notfällen mindestens ein Gerät abgeschaltet werden.

Mittels einer Zwei- oder Mehrpunktsteuerung kann Schaumbildung, insbesondere schnellwachsender Schaum besonders zuverlässig erfasst werden, da einerseits das Schaumniveau innerhalb des Einwegbehälters insbesondere oberhalb des Mediums und andererseits das Vorhandensein von Schaum innerhalb und/oder an Ports, Rohren und/oder Schläuchen erfasst werden kann. So kann es beispielsweise sein, dass der Schaum oberhalb des Mediums effizient mittels eines Antischaum-Mittels bekämpft wird, während der Schaum innerhalb eines Rohres weiter wächst und von dem Antischaum-Mittel nicht erreicht wird. In dem Fall können entsprechende Maßnahmen zur Bekämpfung des Schaums an dieser Position und/oder zum Schutz des Mediums beispielsweise mittels Abschalten zumindest einer Einheit getroffen werden.

Mit Schaumniveau ist hier insbesondere die Entfernung des Schaums zum Behälter-Innenboden gemeint. In anderen Worten ist das Schaumniveau dabei insbesondere die Höhe des Schaums an der höchsten Position vom Boden des Einwegbehälters, wobei der Schaum beispielsweise auf einem fluiden Medium, das in den Einwegbehälter gefüllt ist, stehen kann. In anderen Fällen, in denen Schaum an der Behälter-Innenwandung und/oder an einem Port und/oder in einem Schlauch haftet wird von Vorhandensein von Schaum gesprochen. Die Zwei- oder Mehrpunktsteuerung ist also insbesondere dazu ausgelegt, zuverlässig das Schaumniveau und das Vorhandensein von Schaum an mindestens zwei Positionen der Bioreaktoreinheit zu erfassen. Das Schaumniveau kann beispielsweise mittels zumindest einer ersten Sensoreinheit, die insbesondere auf der Behälterhüllen-Innenwand angeordnet ist, ermittelt oder zumindest abgeschätzt werden, wobei das Vorhandensein von Schaum an einem Port mittels zumindest einer zweiten Sensoreinheit in der Nähe des Ports erfasst werden kann. Alternativ kann eine Sensoreinheit zum Ermitteln eines Schaumniveaus auch von außen an der Behälterhüllen-Außenwand angeordnet sein oder angeordnet werden.

Insbesondere handelt es sich bei einer solchen Sensoreinheit zum Ermitteln eines Schaumniveaus typischerweise um eine Patch-Sensoreinheit, die noch näher beschrieben wird. Solche Sensoreinheiten, die insbesondere zur Kontrolle von Schaumniveaus in einem Einwegbehälter einer Bioreaktoranlage dienen, können auch "Kontrollpflaster" genannt werden kann.

Gleichzeitig erlaubt das System gemäß einer ersten Alternative der beanspruchten Erfindung, das Vorhandensein von Schaum in der unmittelbaren Nähe eines Ports zu erfassen und ggf. zu überwachen. In dem Fall kann an dem Port beispielsweise eine Bogen-Sensoreinheit, die noch näher beschrieben wird, bevorzugt von außen an eine entsprechende Anordnungsposition der Bioreaktoranlage angeordnet werden. Eine solche Sensoreinheit, die nicht zwingend aber bevorzugt eine Bogen-Sensoreinheit darstellt und dazu ausgelegt ist, das Vorhandensein von Schaum insbesondere an Positionen in der Nähe von Ports, Anschlüssen, Schläuchen, Rohren oder ähnlichem zu erfassen, kann auch "Alarmpflaster" genannt werden.

In anderen Worten ist eine weitere Sensoreinheit, insbesondere ein Alarmpflaster, in räumlicher Nähe zu einem oder mehreren Ports und/oder Schläuchen, insbesondere an und/oder bei mindestens einem Abluftschlauch der Bioreaktoranlage angeordnet sein. Bevorzugt wird ein solches Alarmpflaster auf der Außenwand der Bioreaktoranlage angeordnet und unterscheidet sich insbesondere in der Geometrie im Wesentlichen von der Geometrie eines Kontrollpflasters.

Diese Sensoreinheit kann, genau wie alle anderen Sensoreinheiten über einen Transmitter ausgelesen werden, wobei der Transmitter insbesondere in Kontakt, beispielsweise über ein Datenkabel oder über eine drahtlos-Verbindung mit einer Steuersoftware der Bioreaktoranlage steht. Sobald ein Signal des Alarmpflasters einen definierten bzw. vorbestimmten Schwellwert überschreitet, wird eine Notfallprozedur bzw. eine Maßnahme mittels der Steuerung und/oder der Kontrolleinheit der Bioreaktoranlage ausgelöst. Hierbei handelt es sich erfindungsgemäß um eine Abschaltung des Rührers und/oder der Begasung, sowie eine optionale (schnelle) Zugabe von Antischaum-Mittel.

Diese genannte Anordnungskonstellation der Sensoreinheiten an den unterschiedlichen Anordnungspositionen ist besonders vorteilhaft, da somit überwacht werden kann, ob eine Substanz, die dem System zugeführt wird bzw. werden muss, um die Schaumbildung zu verhindern oder zu reduzieren bzw. ein Antischaum-Mittel, effizient an verschiedenen Positionen innerhalb der Bioreaktoranlage wirkt.

Beispielsweise kann das Antischaum-Mittel dem System an einer Stelle der Bioreaktoranlage zugeführt werden, jedoch ggf. nicht an allen Positionen innerhalb der Bioreaktoranlage volle Wirkung entfalten. Insbesondere sind davon Winkel, Ports, Anschlüsse, Rohre und/oder Schläuche betroffen. Sobald ein solcher Zustand von einem Alarmpflaster erfasst wird, kann das Antischaum-Mittel beispielsweise vermehrt und/oder örtlich gezielt eingesetzt werden. In Notfällen, in denen beispielsweise die Schaumbildung durch bloße Hinzugabe von Antischaum-Mittel nicht kontrolliert bzw. reduziert werden kann, kann ein Alarmsignal ausgelöst werden und/oder zumindest ein laufendes Gerät abgeschaltet werden.

Die Verwendung des Alarmpflasters dient bevorzugt auch als zusätzliche Absicherung. Wenn die kontrollierte Zugabe von Antischaummittel basierend auf den Daten eines Kontrollpflasters nicht funktioniert und/oder fehlerhaft ist, beispielsweise durch eine Fehlfunktion des Sensors, einen leeren Vorrat von Antischaummittel und/oder eine Fehlfunktion der Pumpe, kann dies an der Position des Alarmpflasters erfasst werden und entsprechende Gegenmaßnahmen können darauffolgend automatisiert oder manuell eingeleitet werden.

Beispielsweise kann derart eine 2-Punkt Antischaum-Regelung verwirklicht werden, die dazu ausgelegt sein kann, automatisiert die folgenden Schritte auszuführen: Wenn das Vorhandensein von Schaum an einer Anordnungsposition erfasst wird und/oder Schaum, der ein vorbestimmtes Schaumniveau erreicht und/oder übersteigt, erfasst wird, kann infolgedessen eine Pumpe, die dem Innenraum der Bioreaktoranlage ein Antischaum-Mittel zuführt, für eine Dauer aktiviert werden. Darauffolgend kann sich eine Wartezeit für die Wirkung des Antischaum-Mittels anschließen. Wenn daraufhin immer noch Schaum erfasst wird, kann die Pumpe wiederholt für eine Dauer eingeschaltet werden, worauf wiederholt eine Wartezeit folgen kann.

Um ungenaue oder fehlerhafte Messungen zu vermeiden oder effizient auf schnell wachsende Schäume reagieren zu können, ist es vorteilhaft eine Vielzahl von Sensoreinheiten an und/oder in der Bioreaktoranlage anzuordnen, insbesondere in der oberen Hälfte oder im oberen Drittel der Bioreaktoranlage, bevorzugt im oberen Viertel.

Der Vorteil einer kapazitiven Sensoreinheit, die auf dem Elektrodensystem basiert, besteht darin, dass eine Änderung der Permittivität, insbesondere berührungsfrei bzw. *ex-situ*, also außerhalb des Einwegbehälters bzw. durch die Wandung des Einwegbehälters hindurch erfasst bzw. detektiert werden kann. Die kapazitive Sensoreinheit basiert im Wesentlichen auf dem Kondensatorprinzip. Eine Messung kann insbesondere auch durch nicht-leitende Schichten, wie beispielsweise durch Folien und/oder Einwegbehälterhüllen und/oder Behälterwandungen von Einwegbeuteln erfolgen. Beispielsweise kann das Elektrodensystem anhand einer Änderung der Permittivität erfassen, ob ein Schaum oder ein flüssiges Medium, beispielsweise ein wässriges Medium in der unmittelbaren Nähe vorhanden bzw. gegenwärtig ist.

Sofern eine Sensoreinheit innerhalb des Einwegbehälters angeordnet ist oder werden soll, kann das Elektrodensystem mittels einer Folie zur Abschirmung vor dem direkten Kontakt mit einem Medium geschützt werden. In diesem Fall ist es auch vorteilhaft, die Sensoreinheit so auszugestalten, dass sie insbesondere zusammen mit dem Einwegbehälter sterilisiert werden kann.

Das Elektrodensystem kann dabei ein Zwei- oder insbesondere ein Drei-Elektrodensystem darstellen. Ein Zwei-Elektrodensystem weist eine Sensorelektrode und eine Masseelektrode auf und ein Drei-Elektrodensystem weist darüber hinaus noch eine Schutzelektrode auf. Das Elektrodensystem ist dazu ausgelegt, eine Kapazität zu erzeugen bzw. aufzuweisen, die von dem äußeren dielektrischen Feld und damit der vorliegenden Permittivität bestimmt und beeinflusst wird. Eine Änderung der Kapazität weist somit insbesondere auf eine Änderung des äußeren dielektrischen Feldes und somit der vorliegenden Permittivität eines Materials, insbesondere eines Mediums und/oder eines Schaums hin.

Entsprechend kann somit im Wesentlichen ermittelt oder wenigstens abgeschätzt werden, ob ein Medium und/oder ein Schaum in unmittelbarer Nähe an der Anordnungsposition vorliegt oder nicht. Der Begriff "unmittelbare Nähe" weist hier auf eine Distanz hin, innerhalb derer ein Elektrodensystem dazu im Wesentlichen in der Lage ist, eine sich ändernde Permittivität noch erfassen zu können. Die Distanz zwischen der Ebene innerhalb derer das Elektrodensystem zumindest teilweise angeordnet ist und dem Ort der gerade noch messbaren Permittivität liegt im Nahfeld der Elektroden, das heißt je nach Ausführung des Elektrodensystems beispielsweise bei Werten unterhalb von etwa 2 cm, insbesondere unterhalb von etwa 1 cm und bevorzugt unterhalb von etwa 0,5 cm. Insbesondere treten Signale bei Distanzen ab etwa 2 cm auf, robuste Messungen sind dabei insbesondere ab etwa 0,5 cm möglich, beispielsweise bei etwa 0,2 cm oder 0,05 cm.

Bevorzugt wird mindestens eine der Sensoreinheiten mittels eines Transmitters ausgelesen, wobei der Transmitter in einem Kontakt, beispielsweise über ein Kabel und/oder über eine kabellose Verbindung, mit einer Steuersoftware und/oder der Kontrolleinheit der Bioreaktoranlage steht. Sobald das Signal einer Sensoreinheit einen definierten Schwellwert überschreitet kann beispielsweise automatisiert ein AntiSchaummittel in den Bioreaktor gepumpt werden.

Anstatt der kapazitiven Sensoreinheiten können alternativ oder zusätzlich auch konduktive Sensoreinheiten verwendet werden, wobei die Sensoreinheiten in Kontakt mit dem Bioreaktormedium stehen müssen. Konduktive Sensoreinheiten können ausschließlich leitfähige Schäume erfassen.

Bevorzugt weist zumindest eine der kapazitiven Sensoreinheiten einen adhäsiven Klebestreifen mit einer Klebefläche auf, an dem das Elektrodensystem zumindest teilweise angeordnet sein kann, wobei der adhäsive Klebestreifen insbesondere dazu ausgelegt ist, dass die zumindest eine kapazitive Sensoreinheit reversibel an der Bioreaktoranlage angeordnet werden kann.

Ein Klebestreifen erlaubt dem Verwender, die kapazitive Sensoreinheit an einer Behälterhülle bevorzugt von außen aber auch von innen an der Bioreaktoranlage anzuordnen. Insbesondere ist eine kapazitive Sensoreinheit einmalig an einer Behälterhülle anzuordnen und/oder anzukleben, das heißt, die kapazitive Sensoreinheit kann in dem Fall nur an einer Stelle der Behälterhülle zum Einsatz kommen und wird nach der Verwendung zusammen mit dem Einweg-Behälter bzw. dem Einweg-Beutel entsorgt.

Alternativ kann die kapazitive Sensoreinheit mehrfach, also öfter als einmal an einer Oberfläche bzw. Wandung der Bioreaktoranlage, beispielsweise an der Behälterhülle des Einwegbehälters angeordnet werden.

Optional stellt zumindest eine der kapazitiven Sensoreinheiten eine kapazitive Bogen-Sensoreinheit dar, wobei die Form der kapazitiven Bogen-Sensoreinheit zumindest einen Abschnitt eines Bogens aufweist. Beispielsweise kann die Bogen-Sensoreinheit im Wesentlichen die Form eines Halbkreisbogens oder eines Dreiviertelkreisbogens oder eines anderen Anteils eines Kreisbogens aufweisen. Die Bogenform kann auch einem Bogen entsprechend der keinen konstanten Radius aufweist, beispielsweise eines Ellipsenbogens.

Die kapazitive Bogen-Sensoreinheit ermöglicht ein besonders effizientes Anordnen einer Sensoreinheit an Anordnungspositionen, an denen sich das Anordnen komplex gestaltet. So kann es beispielsweise erforderlich oder erwünscht sein, den Schaum an einer Position eines Ports und/oder an einem Schlauch und/oder an einem Rohr erfassen zu können. Die kapazitive Bogen-Sensoreinheit ist so ausgestaltet, dass sie geometrisch um einen Port bzw. eine Öffnung in dem Einwegbehälter zumindest teilweise herumgelegt bzw. zumindest teilweise darum angeordnet werden kann. In anderen Worten kann die kapazitive Bogen-Sensoreinheit einen Port, eine Öffnung, einen Vorsprung, ein Rohr, eine Leitung und/oder einen Schlauch zumindest teilweise umschließen. Zumindest teilweise bedeutet in diesem Zusammenhang insbesondere zu mindestens etwa 50 % eines Bogens, insbesondere eines Kreisbogens. Dies bedeutet, dass die kapazitive Bogen-Sensoreinheit beispielsweise den Bogen eines Halbkreises darstellt. Ein Bogen ist dabei ein Halbkreis mit einer im Wesentlichen zentralen halbkreisförmigen Aussparung, sodass ausreichend Platz für eine Öffnung mit einem Radius r besteht. Der Radius der halbkreisförmigen Aussparung sollte entsprechend einen Radius aufweisen der mindestens dem Radius r der Öffnung entspricht.

Bei Bioreaktoranlagen, die eine Vielzahl von Öffnungen, Ports, Schläuchen und/oder Rohren aufweisen, ist der Einsatz kapazitiver Bogen-Sensoreinheiten von besonderem Vorteil, da relativ platzsparend eine Vielzahl von Sensoreinheiten an der Bioreaktoranlage angeordnet werden können, insbesondere ohne einander flächig überschneiden zu müssen.

Alternativ oder zusätzlich kann diese Sensoreinheit statt eines Bogens auch eine andere Form aufweisen, die einen Port derart nachahmt bzw. entspricht, dass sie zumindest teilweise an dessen äußeren Umfang angepasst ist und diesen zumindest teilweise umschließt. In anderen Worten kann die Sensoreinheit an die Geometrie eines Ports und/oder eines Schlauchs angepasst sein. Beispielsweise kann die Sensoreinheit auch einen im Wesentlichen rechteckigen offenen oder geschlossenen Rahmen, also ein Rechteck mit Aussparung in der Mitte darstellen.

Optional weist die Bioreaktoranlage zumindest einen Port, insbesondere einen kreisförmigen Port zum fluidischen Verbinden des Innenraums des Einwegbehälters mit einem anderen Element und/oder dem Außenraum des Einwegbehälters auf, wobei eine kapazitive Bogen-Sensoreinheit an dem Port angeordnet sein kann und diese dazu ausgelegt ist, den Port zumindest teilweise zu umschließen bzw. zumindest teilweise einzurahmen.

Die Anordnung der kapazitiven Bogen-Sensoreinheit zumindest teilweise um einen Port herum erweist sich als vorteilhaft, da derart möglichst effizient ermittelt werden kann, ob ggf. Schaum an einer Stelle in direkter bzw. unmittelbarer räumlicher Nähe zum Port vorhanden ist. Dies kann der Fall sein, wenn dem Innenraum eines Behälters zwar ein Antischaum-Mittel zugegeben wurde, jedoch nicht alle Stellen, insbesondere an einem Port und/oder innerhalb von Rohren und Schläuchen von dem Antischaum-Mittel erreicht werden und sich somit der Schaum an besonders verwinkelten Positionen innerhalb einer Bioreaktoranlage weiter ausbreiten kann oder zumindest nicht reduziert werden kann.

Es ist in dem Fall möglich, ein Antischaum-Mittel an der Position gezielt einzusetzen, beispielsweise mittels einer dafür vorgesehenen Düse in der Nähe des Schaums, um eine Schaumbildung an dieser Stelle zu vermindern. Alternativ oder zusätzlich kann ein Alarm und/oder eine Notfallausschaltung des gesamten Systems und/oder zumindest eines Elementes des Systems ausgelöst werden und/oder mindestens ein Gerät, beispielsweise eine Pumpe und/oder eine Rührwelle abgestellt werden.

Optional weist die Bioreaktoranlage zumindest einen fluidisch mit dem Port verbundenen Schlauch und/oder ein Rohr auf, wobei die zumindest eine kapazitive Bogen-Sensoreinheit dazu ausgelegt sei kann, den Schlauch und/oder das Rohr zumindest teilweise zu umschließen.

Die Anordnung der kapazitiven Bogen-Sensoreinheit zumindest teilweise um ein Rohr und/oder einen Schlauch herum erweist sich vorteilhaft, da so möglichst effizient ermittelt werden kann, ob ggf. Schaum an einer Stelle innerhalb des Rohrs und/oder des Schlauchs vorhanden ist. Dies kann ggf. der Fall sein, wenn dem Innenraum eines Behälters zwar ein Antischaum-Mittel zugegeben wurde, jedoch nicht alle Stellen, insbesondere innerhalb der Rohre und/oder Schläuche von dem Antischaum-Mittel erreicht werden und sich somit der Schaum an besonders verwinkelten Positionen innerhalb einer Bioreaktoranlage weiter ausbreiten kann oder zumindest nicht reduziert werden kann.

Optional ist zumindest eine der kapazitiven Sensoreinheiten, die bevorzugt eine kapazitive Patch-Sensoreinheit ist, dazu ausgelegt, das Vorhandensein von Schaum, insbesondere ein Schaumniveau bzw. einen Schaumstand in dem Einwegbehälter zu erfassen und bevorzugt kann eine der kapazitiven Sensoreinheiten an einer Anordnungsposition im Innenraum des Einwegbehälters, insbesondere an einer Behälterhüllen-Innenseite reversibel entfernbar oder permanent angeordnet sein, wobei die zumindest ein kapazitive Sensoreinheit bevorzugt sterilisierbar, insbesondere sterilisiert ist.

An Anordnungspositionen, die keine besondere Geometrie der Sensoreinheit erfordern, wie beispielsweise an einer flächigen Behälterhülle, kann eine im Wesentlichen rechteckige kapazitive Patch-Sensoreinheit auf einfache Weise angeordnet werden.

Ein System aus derartigen Sensoreinheiten kann zwei, drei oder mehr Sensoreinheiten aufweisen. Dies ist vorteilhaft, um das Schaumniveau oder den Füllstand innerhalb des Einwegbehälters erfassen zu können. Dazu können die Sensoreinheiten an der Behälterhüllen-Innenseite angeordnet sein. In dem Fall ist es vorteilhaft, die Sensoreinheiten zuvor zu sterilisieren, sodass ein Medium, das in den Einwegbehälter gefüllt wurde, vor Kontaminationen geschützt werden kann. Alternativ kann die Sensoreinheit auch an der Behälterhüllen-Außenseite des Einwegbehälters angeordnet werden. Dies hat den Vorteil, dass auf vorheriges Sterilisieren der Sensoreinheiten verzichtet werden kann, da die Behälterhülle das Medium hinsichtlich eines direkten physischen Kontaktes zur Sensoreinheit abschirmt. Zusätzlich besteht ein Vorteil darin, dass ein Endverbraucher selbst entscheiden kann, wie viele Sensoreinheiten an der Bioreaktoranlage angeordnet werden sollen und/oder an welchen Anordnungspositionen die Sensoreinheiten angeordnet werden sollen.

Sofern die Sensoreinheiten reversibel angeordnet werden können, ist es möglich, diese nachträglich noch einmal an anderen Positionen oder gar an anderen Bioreaktoranlagen anzuordnen, wenn beispielsweise ein Einwegbehälter entsorgt wird.

Der Schaumstand bzw. das Schaumniveau ist insbesondere der Abstand zwischen dem Behälterboden und dem höchsten Punkt des Schaums. Dabei kann der Schaum beispielsweise auf einem Medium stehen.

Eine solche Sensoreinheit, insbesondere eine Patch-Sensoreinheit, die derartig angeordnet ist, kann auch "Kontrollpflaster" genannt werden, da diese zur Kontrolle eines im Wesentlichen regulären oder üblich zu erwartenden Zustandes der Befüllung dienen soll, wobei noch keine kritische Situation bzw. keine Notfall-Situation besteht.

Diesbezüglich kann es auch Sensoreinheiten, insbesondere Patch-Sensoreinheiten geben, die an relativ hohen Anordnungspositionen innerhalb oder außerhalb der Bioreaktoranlage angeordnet werden, um einen besonders hohes nicht zwingend zu erwartendes Schaumniveau erfassen zu können. Beispielsweise kann eine solche Sensoreinheit direkt unterhalb einer Einwegbehälter-Decke oder Behälter-Innendecke angeordnet sein, um erfassen zu können, wenn ein Schaum die Decke des Behälters erreicht. Eine solche Situation, in der der Schaum ein hohes Niveau, beispielsweise ein vorbestimmtes Level erreicht oder überschreitet, kann ggf. als Notfall-Situation eingestuft werden und es kann erwünscht sein, dass in einem solchen Fall eine oder mehrere Maßnahmen getroffen werden.

Optional kann zumindest eine der kapazitiven Sensoreinheiten, insbesondere eine kapazitive Bogen-Sensoreinheit an einer Anordnungsposition außerhalb des Einwegbehälters, insbesondere an einer Behälterhüllen-Außenseite und/oder an einem Port und/oder an einem Schlauch bzw. Rohr reversibel entfernbar oder permanent angeordnet werden oder eine solche kapazitive Bogen-Sensoreinheit ist bereits daran angeordnet.

Die kapazitive Bogen-Sensoreinheit kann insbesondere als "Alarmpflaster" agieren bzw. eingesetzt werden, da sie dazu ausgelegt ist, Vorliegen von Schaum an verwinkelten und insbesondere an hohen Positionen innerhalb der Bioreaktoranlage zu erfassen. Ggf. kann ein Interesse daran bestehen, die Schaumbildung an solchen Positionen, wie beispielsweise innerhalb eines Rohres und/oder an einem Port zu verhindern. Es kann auch sein, dass dem Einwegbehälter bereits ein Antischaum-Mittel zugefügt wurde, jedoch sich die Wirkung nicht im selben Maße innerhalb eines Rohres entfalten kann. Dazu eignet es sich, auch an Ports und/oder Rohren und/oder Schläuchen Sensoreinheiten anzuordnen.

Eine solche Situation, in der Schaum an einem Port vorliegt, kann ggf. als Notfall eingestuft werden und es kann erwünscht sein, dass in einem solchen Fall Maßnahmen getroffen werden. Eine Sensoreinheit, die ausgelegt ist, einen solchen Notfall zu erfassen, kann auch "Alarmpflaster" genannt werden.

Das Anordnen von kapazitiven Bogen-Sensoreinheiten hat den Vorteil, dass besonders platzsparend und effizient die Schaumbildung an verwinkelten Positionen erfasst werden kann.

Vorzugsweise umfasst das System die Kontrolleinheit. Die Kontrolleinheit kann beispielsweise eine Recheneinheit umfassen, die erfasste Daten zum Schaumniveau oder zum Vorliegen von Schaum von mindestens einer der Sensoreinheiten empfängt, verwertet und/oder weiterleitet. Die Kontrolleinheit kann beispielsweise auch eine Steuer- und Regeleinheit umfassen, die beispielsweise von der Recheneinheit Daten empfangen kann. Insbesondere kann die Steuer- und Regeleinheit eine Maßnahme auslösen, wenn ein vorbestimmter Zustand beispielsweise ein Notfallzustand erfasst wurde. Vorzugsweise kann die Steuer- und Regeleinheit einen Zusatz von einem Antischaum-Mittel auslösen, das dazu geeignet ist, die Schaum oder Produktion von Schaum zu reduzieren oder zu verhindern.

Optional ist die Kontrolleinheit dazu ausgelegt, einen Alarm auszulösen, wenn das Vorhandensein von Schaum an einer Anordnungsposition im Innenraum des Einwegbehälters mittels einer kapazitiven Sensoreinheit, insbesondere mittels einer kapazitiven Patch- Sensoreinheit erfasst wird und/oder wenn das Vorhandensein von Schaum an einer Anordnungsposition außerhalb des Einwegbehälters mittels einer kapazitiven Sensoreinheit, insbesondere mittels einer kapazitiven Bogen-Sensoreinheit erfasst wird.

Optional ist die Kontrolleinheit dazu ausgelegt, ein Notfall-Aus auszulösen bzw. mindestens ein Gerät abzuschalten, wenn das Vorhandensein von Schaum mittels einer kapazitiven Sensoreinheit, insbesondere mittels einer kapazitiven Bogen-Sensoreinheit erfasst wird an einer Anordnungsposition außerhalb des Einwegbehälters, insbesondere benachbart zu bzw. in der unmittelbaren Nähe eines Ports und/oder eines Schlauchs und/oder eines Rohrs.

Optional ist die Kontrolleinheit dazu ausgelegt, dem Innenraum des Einwegbehälters zum Steuern und/oder Regeln der Schaumbildung eine Substanz zuzuführen, die dazu ausgelegt ist, eine Schaumbildung zu verhindern oder zumindest zu vermindern, wenn ein vorbestimmter Schaum-Niveau mittels zumindest einer kapazitiven Sensoreinheit an einer Anordnungsposition im Innenraum des Einwegbehälters und/oder an einer Anordnungsposition außerhalb des Einwegbehälters erfasst wird.

Optional weist zumindest eine der Anordnungspositionen im Innenraum des Einwegbehälters und/oder außerhalb des Einwegbehälters eine vormarkierte Anordnungsposition auf und eignet sich zum Andeuten der jeweiligen Anordnungsposition für den Verwender und zum vereinfachten Anordnen der jeweiligen kapazitiven Sensoreinheit.

Optional übermittelt die kapazitive Sensoreinheit jeweils mittels eines Datenkabels bzw. mittels eines Datenkabel-Systems Daten zum Vorhandensein von Schaum an die Kontrolleinheit, insbesondere an eine Recheneinheit der Kontrolleinheit.

Erfindungsgemäß weist die Bioreaktoranlage ferner einen Abluftport auf zum fluidischen Verbinden des Behälter-Innenraums mit dem Außenraum und/oder einer weiteren Komponente und insbesondere zum Abführen von Luft aus der Bioreaktoranlage, insbesondere aus dem Einwegbehälter. Gemäß einer zweiten Alternative der beanspruchten Erfindung weist die Bioreaktoranlage ferner einen Abluftschlauch auf, wobei der Abluftschlauch fluidisch mit dem Abluftport verbunden ist. Optional weist die Bioreaktoranlage ferner einen Filter, insbesondere einen Sterilfilter auf, der an dem Abluftschlauch angeordnet ist und ein Medium, insbesondere austretende Luft filtern kann, wenn diese ggf. durch den Abluftschlauch tritt. Optional weist die Bioreaktoranlage ferner einen Port für einen Ober-Zugang und/oder Ober-Ablauf und bevorzugt ein damit fluidisch verbundener Schlauch und/oder ein Rohr auf.

Erfindungsgemäß stellt die Anordnungsposition an der Behälterhüllen-Außenseite zumindest eine der folgenden Anordnungspositionen dar:
- Anordnungsposition in unmittelbarer Nähe des Abluftports;
- Anordnungsposition an einem Abluftschlauch der Bioreaktoranlage, wobei der Abluftschlauch fluidisch mit dem Abluftport verbunden ist und sich die Anordnungsposition in unmittelbarer Nähe des Abluftports befindet;
- Anordnungsposition an dem Abluftschlauch der Bioreaktoranlage in unmittelbarer Nähe eines Filters des Abluftschlauchs, insbesondere zwischen dem Abluftport und dem Filter.

In allen Fällen ist mit dem Begriff "knapp oberhalb" insbesondere eine Distanz zwischen der jeweiligen genannten Höhe und der Unterkante der Sensoreinheit gemeint, die zwischen etwa 0,2 cm und etwa 10 cm, insbesondere zwischen 0,5 cm und 5 cm und bevorzugt zwischen etwa 1 cm und 3 cm liegt.

In allen Fällen ist mit dem Begriff "in unmittelbarer Nähe" bzw. "in unmittelbarer Umgebung" oder "im unmittelbaren Umfeld" insbesondere eine Distanz zwischen einer Kante eines Elements und einer Kante der Sensoreinheit, insbesondere zwei Kanten mit der kürzesten Distanz zueinander, gemeint, die zwischen etwa 0,2 cm und etwa 10 cm, insbesondere zwischen 0,5 cm und 5 cm und bevorzugt zwischen etwa 1 cm und 3 cm liegt.

Die Anordnungspositionen der Sensoreinheiten in der Nähe oder in etwa auf Höhe oder knapp unterhalb eines Abluftschlauchs können vorteilhaft dazu dienen, einen Zustand zu erfassen, in dem ein Schaum, insbesondere ein schnell-wachsender Schaum in den Abluftschlauch hineinwächst bzw. hineintritt und diesen ggf. verstopft, sodass die Abluft nicht mehr zuverlässig austreten kann oder Filter an dem Abluftschlauch mit dem Schaum kontaminiert werden. In einem solchen Fall kann ein Notfall vorliegen und somit können sofortige Maßnahmen beispielsweise automatisiert und/oder manuell eingeleitet werden, die ein weiteres Anwachsen von Schaum verhindern können. Beispielsweise kann eine Rührwelle abgestellt werden und/oder ein Teil des Mediums kann abgelassen werden und/oder ein Antischaum-Mittel kann zugeführt werden und/oder ein Port, beispielsweise der Abluftport kann mittels eines Ventils verschlossen werden.

Optional stellt die Anordnungsposition an der Behälterhüllen-Innenseite zumindest eine der folgenden Anordnungspositionen dar:
- Anordnungsposition in der Nähe, bevorzugt auf einer Höhe knapp oberhalb einer Höhe eines vorbestimmten maximalen Füllstandes des Einwegbehälters, insbesondere oberhalb einer Grenzlinie des vorbestimmten maximalen Füllstandes;
- Anordnungsposition in der Nähe, bevorzugt auf einer Höhe knapp oberhalb einer Höhe eines vorbestimmten minimalen Füllstandes des Einwegbehälters, insbesondere einer Grenzlinie des vorbestimmten minimalen Füllstandes;
- Anordnungsposition auf einer Höhe zwischen der Höhe des vorbestimmten minimalen Füllstandes und der Höhe des vorbestimmten maximalen Füllstandes, insbesondere zwischen einer Grenzlinie des vorbestimmten minimalen Füllstandes und einer Grenzlinie des vorbestimmten maximalen Füllstandes.

Die zuvor genannten Bereichsangaben sind insbesondere auch in diesem Fall anzuwenden bei der Deutung der Ausdrücke "in unmittelbarer Nähe" und "knapp oberhalb".

Je nach Größe der Bioreaktoranlage und/oder des Behälters liegt der Abstand zwischen einer Sensoreinheit und dem entsprechenden vorbestimmten Füllstand bevorzugt zwischen mindestens etwa 2 cm und etwa 9 cm. Mit anderen Worten ist eine Sensoreinheit bevorzugt zwischen etwa 2 cm und etwa 9 cm oberhalb des vorbestimmten minimalen und/oder des maximalen Füllstandes angeordnet.

Die genannten Anordnungspositionen der Sensoreinheiten sind insbesondere von Vorteil zur Erfassung von Schaumniveaus, mit denen üblicherweise zu rechnen ist bzw. im normalen Betrieb der Bioreaktoranlage auftreten können. Die Sensoreinheiten an den genannten Anordnungspositionen können zwar auch Notfallsituationen erfassen, insbesondere die Sensoreinheit, die knapp oberhalb eines vorbestimmten maximalen Füllstandes angeordnet sein kann, jedoch sind die Sensoreinheiten insbesondere dazu ausgelegt Schaumniveaus zu erfassen, welche noch keine Notfallsituationen erzeugen.

Eine Kombination aus solchen Sensoreinheiten, welche dazu angeordnet sind, übliche Schaumniveaus zu erfassen und solchen Sensoreinheiten, welche dazu angeordnet sind, Notfall-Situationen zu erfassen, kann besonders von Vorteil sein, da einerseits die Situation innerhalb des Einwegbehälters überwacht werden kann und andererseits das Vorhandensein von Schaum an einem Port und/oder in einem Rohr und/oder Schlauch erfasst werden kann. So kann ein möglichst umfassendes Bild der Situation erfasst werden, wenn sich beispielsweise der Schaum innerhalb des Behälters bereits auf Hinzufügen eines Antischaum-Mittels im Wesentlichen zurückbildet, jedoch innerhalb eines Rohres sich die Wirkung des Antischaum-Mittels noch nicht entfalten kann und der Schaum noch vorhanden ist oder gar wächst. Somit kann beispielsweise wiederholt Antischaum-Mittel, insbesondere gezielt an einer Position, an der Schaum erfasst wird, zugeführt werden. Darüber hinaus kann die Steuerung des Systems mittels mindestens zweier Sensoreinheiten erfolgen. Bevorzugt kommt eine Vielzahl von Patch-Sensoreinheiten zum Einsatz. So können insbesondere z.B. fünf, sechs, sieben, acht, neun oder 10 Patch-Sensoreinheiten an der Bioreaktoranlage angeordnet werden. Alternativ können auch mehr oder weniger Patch-Sensoreinheiten an der Bioreaktoranlage angeordnet werden.

Die Patch-Sensoreinheit kann dabei ein Kontrollpflaster darstellen und die Bogen-Sensoreinheit kann ein Alarmpflaster darstellen. Insbesondere kann ein Alarmpflaster an einem Port eine Maßnahme, wie ein interlock Notfall-Aus für mindestens ein Gerät auslösen, insbesondere wenn die Steuerung einer Antischaum-Mittelzugabe ausfällt.

Optional weist das System die folgenden Anordnungspositionen auf, an denen jeweils mindestens ein Sensor angeordnet sein kann oder angeordnet ist:
- Anordnungsposition, insbesondere für ein Kontrollpflaster, in der Nähe, bevorzugt auf einer Höhe knapp oberhalb einer Höhe eines vorbestimmten maximalen Füllstandes des Einwegbehälters, insbesondere oberhalb einer Grenzlinie des vorbestimmten maximalen Füllstandes;
- Anordnungsposition, insbesondere für ein Alarmpflaster, in unmittelbarer Nähe des Abluftports.

Insbesondere kann in diesem System das Signal von dem Kontrollpflaster die Antischaum-Zugabe regeln. Im Normalfall löst das Alarmpflaster keine Maßnahme aus, wenn genügend Antischaum-Mittel zugegeben wird, um eine starkes und/oder schnelles Schäumen zu verhindern. Das Alarmpflaster dient insbesondere als "zweite Instanz", um bei einem Ausfall der Antischaum-Mittel-Zugabe, beispielsweise ausgelöst durch eine Fehlfunktion des Kontrollpflasters oder bei nicht ausreichender Wirksamkeit des Antischaum-Mittels vor der Verblockung des Abluftfilters eingreifen zu können. Ein Auslösen des Alarmpflasters sorgt insbesondere für eine Abschaltung schaumfördernder Aktuatoren im Bioreaktor, wie beispielsweise eine Begasungseinheit und/oder eine Rühreinheit. Hierfür ist eine Kombination eines beliebigen Kontrollpflasters mit einem beliebigen Alarmpflaster vorteilhaft.

Optional weist das System die folgenden Anordnungspositionen auf, an denen jeweils mindestens eine kapazitive Sensoreinheit angeordnet sein kann oder angeordnet ist:
- Anordnungsposition, insbesondere für ein Kontrollpflaster, in der Nähe, bevorzugt auf einer Höhe knapp oberhalb einer Höhe eines vorbestimmten maximalen Füllstandes des Einwegbehälters, insbesondere oberhalb einer Grenzlinie des vorbestimmten maximalen Füllstandes;
- Anordnungsposition, insbesondere für ein erstes Alarmpflaster, in unmittelbarer Nähe des Abluftports;
- Anordnungsposition, insbesondere für ein zweites Alarmpflaster, an dem Abluftschlauch der Bioreaktoranlage in unmittelbarer Nähe eines Filters des Abluftschlauchs, insbesondere zwischen dem Abluftport und dem Filter.

Für den Fall, dass sowohl das Kontrollpflaster als auch das erste Alarmpflaster ausfallen kann immer noch über ein Signal des zweiten Alarmpflasters ein Alarm ausgelöst werden und/oder eine Abschaltung schaumfördernder Aktuatoren im Bioreaktor erreicht werden.

Optional weist das System die folgenden Anordnungspositionen an der Behälterhülle, beispielsweise an der Behälterhüllen-Innenseite auf, an denen jeweils mindestens eine kapazitive Sensoreinheit, insbesondere mindestens eine kapazitive Patch-Sensoreinheit angeordnet sein kann oder angeordnet ist:
- Anordnungsposition, insbesondere für ein erstes Kontrollpflaster, in der Nähe, bevorzugt auf einer Höhe knapp oberhalb einer Höhe eines vorbestimmten maximalen Füllstandes des Einwegbehälters, insbesondere oberhalb einer Grenzlinie des vorbestimmten maximalen Füllstandes;
- Anordnungsposition, insbesondere für ein zweites Kontrollpflaster, in der Nähe, bevorzugt auf einer Höhe knapp oberhalb einer Höhe eines vorbestimmten minimalen Füllstandes des Einwegbehälters, insbesondere einer Grenzlinie des vorbestimmten minimalen Füllstandes;
- Anordnungsposition, insbesondere für ein drittes Kontrollpflaster, auf einer Höhe zwischen der Höhe des vorbestimmten minimalen Füllstandes und der Höhe des vorbestimmten maximalen Füllstandes, insbesondere zwischen einer Grenzlinie des vorbestimmten minimalen Füllstandes und einer Grenzlinie des vorbestimmten maximalen Füllstandes.

Mit dem genannten System kann insbesondere die Zugabe von Antischaum-Mittel in verschiedenen Phasen des Bioprozesses gesteuert bzw. geregelt werden. Dies ist beispielsweise vorteilhaft wenn zu Beginn des Bioprozesses mit einem relativ geringen Füllvolumen gestartet wird, das insbesondere im Laufe der Kultivierung durch Zugabe einer Nährsubstanz gesteigert wird. Sobald der Medienfüllstand die Höhe einer Sensoreinheit erreicht, kann diese in der Automatisierungssoftware abgeschaltet werden, um insbesondere eine im Wesentlichen dauerhafte Zugabe von Antischaum-Mittel zu verhindern. Prinzipiell kann unter Umständen, beispielsweise bei einer speziellen Einstellung, insbesondere eine Einstellung auf eine vergleichsweise geringe Sensitivität der Sensoreinheit, diese auch im Wesentlichen zur Detektion des Medienfüllstands genutzt werden. Bevorzugt kann auch ein oder können mehrere zusätzliche der genannten kapazitiven Patch-Sensoreinheiten verwendet werden.

Bevorzugt ist eine der kapazitiven Sensoreinheiten - unabhängig von deren Form - dazu ausgelegt, abgeschaltet bzw. inaktiviert zu werden. Insbesondere ist eine kapazitive Sensoreinheit, die an einer Anordnungsposition im unteren Drittel der Bioreaktoranlage angeordnet ist, dazu ausgelegt abgeschaltet zu werden.

Insbesondere ist das System dazu ausgelegt, eine der kapazitiven Sensoreinheiten abzuschalten. Insbesondere ist das System dazu ausgelegt, eine kapazitive Sensoreinheit, die an einer Anordnungsposition im unteren Drittel der Bioreaktoranlage angeordnet ist, abzuschalten.

Es besteht insbesondere dann ein Vorteil darin, eine kapazitive Sensoreinheit abschalten zu können, wenn die Bioreaktoranlage mit einem Medium befüllt wird, sodass der Füllstand auf einem höheren Niveau liegt als die Anordnungsposition der kapazitiven Sensoreinheit. Die kapazitive Sensoreinheit kann in diesem Fall keinen Schaum erfassen sondern lediglich die Anwesenheit des Mediums, was zu einem unerwünschten Ergebnis bei der Auswertung der Daten führen kann. Darüber hinaus ist es ohnehin ggf. nicht nötig, die kapazitive Sensoreinheit aktiv messend zu belassen, da diese in diesem Zustand keinen Schaum erfassen kann.

Gemäß einem weiteren Aspekt ist eine kapazitive Bogen-Sensoreinheit zum Erfassen eines Vorhandenseins von Schaum eines Mediums, insbesondere in einer Bioreaktoranlage ausgelegt, wobei die kapazitive Bogen-Sensoreinheit zumindest teilweise eine Form aufweist, die zumindest einem Abschnitt eines Bogens, insbesondere eines Kreisbogens entspricht und dazu ausgelegt ist, einen Port und/oder einen Schlauch und/oder ein Rohr einer Bioreaktoranlage zumindest teilweise zu umschließen.

Die Messfläche der kapazitiven Bogen-Sensoreinheit, die insbesondere als Alarmpflaster verwendet wird, ist geometrisch im Wesentlichen so gestaltet, dass sie möglichst nah an die Anschlüsse der Abluftschläuche, beispielsweise zumindest teilweise um einen Port herum angebracht werden kann oder bereits angebracht ist. Dabei kann die kapazitive Bogen-Sensoreinheit den äußeren Umfang der Anschlüsse, beispielsweise einen runden Port zu einem Großteil, insbesondere zu mehr als 50 % umschließen, beispielsweise zu etwa 50 bis 100 %, insbesondere zu etwa 55 bis 90% und bevorzugt zu etwa 60 bis 70%.

Die kapazitive Bogen-Sensoreinheit wird dabei so bevorzugt von außen an der Bioreaktoranlage angebracht, dass die Messrichtung der kapazitiven Sensoreinheit in Richtung des Innenraums der Bioreaktoranlage zeigt, beispielsweise in die Richtung des Innenraums des Einwegbehälters oder in die Richtung des Innenraums eines Schlauchs oder eines Rohrs, um so das Vorhandensein oder nicht Vorhandensein des sich ggf. bildenden Schaums detektieren bzw. erfassen zu können. Die Geometrie der kapazitiven Bogen-Sensoreinheit hat den wesentlichen Vorteil einer verbesserten und schnelleren Schaumerkennung und zwar auch an Anordnungspositionen, die für Sensoreinheiten anderer Geometrien ungünstig liegen. Insbesondere hinsichtlich der stark begrenzten Fläche der Behälterhülle bzw. des Film eines Einwegbeutels bzw. der Bioreaktorwand und der Vielzahl von Elementen, wie einer Rührwelle, Ports, Anschlüssen und Schläuchen bei Einwegsystemen oder Einwegbehältern von Bioreaktoren, erweist sich die Geometrie der kapazitiven Bogen-Sensoreinheit als besonders vorteilhaft, platzsparend und effizient. Dies ist insbesondere dann der Fall, wenn das Behältervolumen kleiner als 100 L ist, beispielsweise etwa 70 L, da in dem Fall die Oberfläche der Bioreaktoranlage besonders wenig Platz zur Anordnung einer Vielzahl von Elementen bietet. Auch kann es vorteilhaft eine hohe Anzahl an Sensoreinheiten an einer kleineren Bioreaktoranlage anzuordnen, da in dem Fall kritische Bereiche von einem Schaum ggf. schneller erreicht werden können wegen des geringen Volumens.

Bevorzugt umfasst die kapazitive Bogen-Sensoreinheit zumindest ein Elektrodensystem mit einer Sensorelektrode und einer Schutzelektrode und insbesondere mit einer Masseelektrode zum kapazitiven Messen an einer der Anordnungspositionen auf der Behälterhüllen-Innenseite und/oder auf der Behälterhüllen-Außenseite der Bioreaktoranlage und zum Erfassen des Vorhandenseins von Schaum an der Anordnungsposition auf Basis der kapazitiven Messung.

Der Vorteil einer kapazitiven Bogen-Sensoreinheit, die auf dem Elektrodensystem basiert, besteht darin, dass eine Änderung der Permittivität erfasst bzw. detektiert werden kann. Dies kann insbesondere auch durch nicht-leitende Schichten, wie beispielsweise durch Folien und/oder Einwegbehälterhüllen und/oder Behälterwandungen von Einwegbeuteln erfolgen. Beispielsweise kann das Elektrodensystem anhand einer Änderung der Permittivität erfassen, ob ein Schaum oder ein flüssiges Medium, beispielsweise ein wässriges Medium in der unmittelbaren Nähe vorhanden ist bzw. präsent ist.

Das Elektrodensystem kann ein Zwei- oder insbesondere ein Drei-Elektrodensystem darstellen. Ein Zwei-Elektrodensystem umfasst insbesondere eine Sensorelektrode und eine Schutzelektrode und ein Drei-Elektrodensystem umfasst darüber hinaus eine Masseelektrode. Die Masseelektrode dient dazu, das Elektrodensystem und damit die kapazitive Sensoreinheit vor hohen Ladungen gegenüber der Masse bzw. Erde zu schützen und entsprechend zu erden. Die Sensorelektrode und die Schutzelektrode sind dazu ausgelegt, eine Kapazität zu erzeugen, die von dem äußeren dielektrischen Feld und damit der vorliegenden Permittivität bestimmt wird. Eine Änderung der Kapazität weist somit ggf. auf eine Änderung des äußeren dielektrischen Feldes und somit der vorliegenden Permittivität eines Materials, insbesondere eines Mediums und eines Schaums hin. Entsprechend kann somit im Wesentlichen ermittelt werden, ob ein Medium und/oder ein Schaum in unmittelbarer Nähe an der Anordnungsposition vorliegt oder nicht. Der Begriff "unmittelbare Nähe" weist hier insbesondere auf eine Distanz hin, innerhalb derer ein Elektrodensystem dazu in der Lage ist, eine sich ändernde Permittivität noch erfassen zu können. Die Distanz zwischen der Ebene bzw. der Fläche innerhalb derer das Elektrodensystem im Wesentlichen angeordnet ist bzw. vorliegt und der gerade noch zu messenden Permittivität liegt je nach Ausführung des Elektrodensystems ggf. bei Werten unterhalb von etwa 1 cm, insbesondere unterhalb von etwa 0,3 cm.

Bevorzugt imitiert bzw. empfindet zumindest ein Abschnitt zumindest einer der Elektroden des Elektrodensystems die Form der kapazitiven Bogen-Sensoreinheit zumindest teilweise nach, wobei die kapazitive Bogen-Sensoreinheit bevorzugt einen adhäsiven Klebestreifen aufweist und die Elektroden optional zumindest teilweise in unterschiedlichen Schichten bzw. Ebenen auf dem adhäsiven Klebestreifen angeordnet sind.

Eine Anordnung des Elektrodensystems, bei der zumindest ein Abschnitt zumindest einer der Elektroden die Form der kapazitiven Bogen-Sensoreinheit nachempfindet ist besonders platzsparend und erlaubt insbesondere eine besonders effiziente Messung bzw. Detektion bzw. Erfassung von Schaum. Eine geschichtete Anordnung des Elektrodensystems kann vorteilhafterweise auch bzw. zusätzlich besonders platzsparend sein.

Die zuvor genannten Ausführungsformen und Merkmale des Systems finden in entsprechender Weise auch in den folgenden Verfahren gemäß einem der Aspekte oder gemäß einer der Ausführungsformen Anwendung. Die daraus resultierenden Vorteile, die insbesondere schon erwähnt wurden ergeben sich ebenfalls für das Verfahren gemäß des Aspekts und insbesondere gemäß einer der möglichen Ausführungsformen.

Bevorzugt weist die kapazitive Bogen-Sensoreinheit Folgendes auf:
- ein Feld, insbesondere einen Bogenabschnitt zum Anbringen an einer der Anordnungspositionen;
- eine Konnektierungslippe, zum Verbinden, insbesondere zum elektrischen Verbinden eines Netzanschlusses und/oder eines Datenanschlusses mit dem Elektrodensystem; und
- einen Abstandshalter, der dazu ausgelegt ist, die Konnektierungslippe zumindest teilweise zu umschließen. Der Abstandshalter sollte ein Material mit einer Dielektrizitätszahl kleiner *εᵣ* aufweisen und dazu ausgelegt sein Fremdkörper ausreichend auf Abstand zu halten, sodass diese nicht relevant in das durch die Konnektierungslippe erzeugte Feld eindringen können.

Bevorzugt weist eine Sensoreinheit, insbesondere eine Bogen- oder eine Patch-Sensoreinheit, einen Abstandshalter auf, der dazu geeignet ist, Störsignale zu vermindern oder gar zu vermeiden. Der Abstandshalter kann insbesondere eine Dicke von etwa 0,2 mm bis etwa 3 mm, bevorzugt von etwa 0,8 mm bis etwa 1,3 mm und besonders bevorzugt von etwa 1 mm aufweisen. Eine Dicke von etwa 1 mm erlaubt eine hohe Flexibilität der Konnektierungslippe 28 und sichert gleichzeitig einen ausreichenden Abstand von möglichen Fremdkörpern um Signaleinkopplung in den sensitiven Bereichen der Konnektierungslippe zu verhindern.

Bevorzugt ist die Konnektierungslippe eine im Wesentlichen längliche und insbesondere flexible Konnektierungslippe, die sich derart bewegen und/oder verbiegen lässt, dass eine Anschlusseinheit, wie insbesondere ein Netzanschluss bzw. eine Versorgungsspannung und/oder ein Datenanschluss von verschiedenen Richtungen mittels der Konnektierungslippe an der Sensoreinheit angeordnet bzw. angeschlossen werden kann. Dies erlaubt dem Verwender einen besonders einfachen Zugang beim Anschluss der Sensoreinheit, da die längliche flexible Konnektierungslippe von mehreren Seiten kontaktiert werden kann, indem sie zu dem Verwender hingebogen wird.

Bevorzugt weist der Abstandshalter einen abschirmenden Kunststoff, insbesondere Polypropylen auf, der bevorzugt zumindest teilweise eine Dicke von etwa 1mm aufweist.

Eine Konnektierungslippe mit Abstandshalter erlaubt dem Verwender, die kapazitive Bogen-Sensoreinheit besonders einfach anzuschließen und dabei keine falsch-positiv Signale zur erfassen, die möglicherweise durch Fremdkörper mit hohem *εᵣ* in der Nähe der Konnektierungslippe erzeugt werden.

Insbesondere kann der Abstandshalter vermindern oder verhindern, dass die kapazitive Sensoreinheit Änderungen in der Permittivität und/oder der Kapazität im unmittelbaren Umfeld der Konnektierungslippe erfasst werden und lediglich solche Änderungen im unmittelbaren Umfeld des Bogenabschnitts erfasst werden.

Alternativ kann auch eine kapazitive Patch-Sensoreinheit aufweisen:
- ein Feld, insbesondere ein rechteckiges Feld bzw. Patch zum Anbringen an einer der Anordnungspositionen;
- eine Konnektierungslippe, zum Verbinden, insbesondere zum elektrischen Verbinden eines Netzanschlusses und/oder eines Datenanschlusses mit dem Elektrodensystem; und
- einen Abstandshalter, der dazu ausgelegt ist, die Konnektierungslippe zumindest teilweise zu umschließen.

Gemäß einem weiteren Aspekt weist ein Verfahren zum Erfassen zumindest eines Vorhandenseins von Schaum eines Mediums die Schritte auf:
- Bereitstellen einer Bioreaktoranlage mit zumindest einem Einwegbehälter zum Aufnehmen des Mediums, das den Schaum umfassen kann;
- Anordnen an mindestens zwei Anordnungspositionen der Bioreaktoranlage insbesondere an einer Behälterhüllen-Innenseite und/oder an einer Behälterhüllen-Außenseite der Bioreaktoranlage zumindest zweier kapazitiver Sensoreinheiten, wobei die kapazitiven Sensoreinheiten jeweils zumindest ein Elektrodensystem für eine kapazitive Messung aufweisen;
- Erfassen von Daten betreffend das Vorhandensein von Schaum an den zumindest zwei Messpositionen auf Basis der kapazitiven Messungen; und
- Übermitteln der erfassten Daten betreffend das Vorhandensein von Schaum an zumindest eine Kontrolleinheit zum Kontrollieren insbesondere zum Überwachen, Steuern und/oder Regeln einer Schaumbildung in der Bioreaktoranlage auf deren Basis mittels der kapazitiven Sensor-Einheiten.

Bevorzugt umfasst das Verfahren zumindest einen der Schritte: Überwachen, Steuern und/oder Regeln der Schaumbildung in der Bioreaktoranlage mittels der Kontrolleinheit auf Basis der übermittelten Daten.

Bevorzugt weist das Steuern und/oder Regeln ein Zuführen einer Substanz auf, die dazu ausgelegt ist, eine Schaumbildung zu verhindern oder zumindest zu vermindern, wenn das Vorhandensein von Schaum mittels zumindest einer kapazitiven Sensoreinheit erfasst wird, bevorzugt, wenn das Vorhandensein von Schaum mittels zumindest einer kapazitiven Sensoreinheit an einer Anordnungsposition außerhalb des Einwegbehälters, insbesondere mittels einer kapazitiven Bogen-Sensoreinheit erfasst wird.

Optional weist das Verfahren die Schritte auf:
- Auslösen eines Alarms wenn das Vorhandensein von Schaum mittels zumindest einer kapazitiven Sensoreinheit an einer Anordnungsposition im Innenraum des Einwegbehälters und/oder an einer Anordnungsposition außerhalb des Einwegbehälters erfasst wird; und/oder
- Auslösen eines Notfall-Aus wenn das Vorhandensein von Schaum mittels zumindest eines Sensors an einer Anordnungsposition außerhalb des Einwegbehälters erfasst wird.

Gemäß einem weiteren Aspekt umfasst ein Verfahren zur Montage eines Systems zum Erfassen zumindest eines Vorhandenseins von Schaum eines Mediums in einer Bioreaktoranlage:
- Bereitstellen einer Bioreaktoranlage mit zumindest einem Einwegbehälter zum Aufnehmen des Mediums, das den Schaum umfassen kann;
- Bereitstellen zumindest zweier kapazitiver Sensoreinheiten, wobei die kapazitiven Sensoreinheiten jeweils zumindest ein Elektrodensystem für eine kapazitive Messung aufweisen;
- Anordnen der zumindest zwei kapazitiven Sensoreinheiten an jeweils einer Anordnungsposition der Bioreaktoranlage insbesondere an einer Behälterhüllen-Außenseite der Bioreaktoranlage; und
- Anschließen der zumindest zwei kapazitiven Sensoreinheiten an zumindest eine Kontrolleinheit zum Kontrollieren insbesondere zum Überwachen, Steuern und/oder Regeln einer Schaumbildung in der Bioreaktoranlage auf Basis der kapazitiven Messungen.

Vorzugsweise können Messeigenschaften variiert werden. Dies kann beispielsweise eine variable Sensitivität der Transmitter und/oder der Sensoreinheiten (beispielsweise etwa 5, etwa 10 und/oder etwa 100 pF) betreffen. Darüber hinaus kann eine Sensoreinheit auch an einer Anordnungsposition innerhalb der Bioreaktoranlage angeordnet werden, beispielsweise an einer Rühreinheit, insbesondere an einer Rührwelle, um tiefer in der Bioreaktoranlage messen zu können. Diese bevorzugten Merkmale sind insbesondere vorteilhaft, da in Bioprozessen ggf. unterschiedliche Organismen eingesetzt werden und abhängig von den vorausgesetzten Prozessbedingungen unterschiedliche Schäume entstehen können.

Im Folgenden werden einige Ausführungsbeispiele näher beschrieben, wobei die Erfindung nicht auf die beschriebenen Ausführungsbeispiele beschränkt sein soll. Einzelne Merkmale, die in einer bestimmten Ausführungsform beschrieben werden, können beliebig kombiniert werden, vorausgesetzt, sie schließen einander nicht aus. Darüber hinaus sind verschiedene Merkmale, die in den beispielhaften Ausführungsformen zusammen bereitgestellt werden, nicht als die Erfindung, wie sie in den Ansprüchen definiert ist, einschränkend anzusehen.
**Fig. 1a** ist eine beispielhafte schematische Darstellung einer Patch-Sensoreinheit;
**Fig. 1b** ist eine beispielhafte schematische Darstellung einer Patch-Sensoreinheit mit einem Abstandshalter an einer Konnektierungslippe;
**Fig. 2a** ist eine beispielhafte schematische Darstellung einer Bogen-Sensoreinheit;
**Fig. 2b** ist eine beispielhafte schematische Darstellung einer weiteren Bogen-Sensoreinheit;
**Fig. 3** ist eine schematische Darstellung einer Bioreaktoranlage nach einer Ausführungsform, die schematisch angeschnitten ist, um einen Einblick in den Innenraum des Behälters zu geben;
**Fig. 4** ist eine schematische Darstellung einer Bioreaktoranlage nach einer Ausführungsform mit Ansicht von außen;
**Fig. 5** ist eine realistische Darstellung einer Bioreaktoranlage nach einer Ausführungsform mit Ansicht von außen; und
**Fig. 6** ist eine beispielhafte schematische perspektivische Darstellung einer Patch-Sensoreinheit.

Bioreaktoren bzw. Bioreaktoranlagen sind insbesondere Anlagen mit einem oder mehreren Behältern zur Aufnahme von Medien. Ein Bioreaktor ist in der Regel dazu ausgelegt, biochemische und/oder chemische Prozesse zu überwachen, zu steuern und/oder zu regeln. Bioreaktoren werden insbesondere als Fermenter zum Kultivieren von Mikroorganismen, Zellen, Einzellern und/oder Mehrzellern eingesetzt. Ein Bioreaktor kann insbesondere als Einwegbehälter ausgestaltet sein und weitere Elemente eines Bioreaktors umfassen. Beispielsweise kann ein Beutel einen wesentlichen Bestandteil eines Bioreaktors darstellen, wobei der Beutel von einem Edelstahlteilgerüst gehalten werden kann.

Der Einwegbehälter (single use bzw. SU Behälter) dient insbesondere zur einmaligen Verwendung für einen oder mehrere hintereinander ablaufende biochemische Prozesse. Nach der Verwendung kann ein Einwegbehälter entsorgt werden, ohne ggf. zuvor auf potentiell aufwendige Weise gereinigt werden zu müssen.

Medien, die in Bioreaktoren gefüllt werden und/oder Zwischenprodukte dieser Medien können unter Umständen einen Schaum bilden, insbesondere bei Rührvorgängen oder während eines oder während mehrerer Prozesse. Schaumbildung ist aus mehreren Gründen problematisch. Einerseits kann Schaum Ein-/Ausgänge und/oder Ports blockieren und andererseits kann sich Schaum negativ auf die Prozesse innerhalb des Bioreaktors auswirken. Daher ist die Überwachung von Bioreaktoren und Bioreaktoranlagen hinsichtlich der Erzeugung von Schaum wesentlich. Insbesondere ist es vorteilhaft, schnell-wachsende Schäume zu überwachen, da diese in kurzen Zeitspannen entstehen und ggf. wertvolle Medien in einem Bioreaktor durch Störungen durch Schaum gefährdet werden können. Bei Fermentationsprozessen besteht auch insbesondere das Risiko, dass nicht erkannter Schaum beispielsweise in den Abluftfilter einer Bioreaktoranlage wächst bzw. hineingelangt und dort einen Filter blockiert. Dies kann ggf. in einem sehr schnellen Druckanstieg in der Bioreaktoranlage führen, beispielsweise eine Regelung des Sauerstoffgehalts des flüssigen Mediums verhindern kann. Als Resultat dessen kann es vorkommen, dass die gesamte Medienfüllung der Bioreaktoranlage verworfen werden muss. Dies kann im schlimmsten Fall zu Produktionsausfällen und hohen Kosten führen. Deshalb ist es wichtig, Schaumbildung effizient und kontinuierlich zu erfassen.

Zu diesem Zweck können Sensoreinheiten, insbesondere kapazitive Sensoreinheiten an und/oder in einer Bioreaktoranlage angeordnet werden. Dabei kommt mindestens eine Zweipunktüberwachung oder sogar eine Mehrpunktüberwachung zum Einsatz, wobei die Zweipunkt- oder Mehrpunktüberwachung jeweils zwei oder mehrere kapazitive Sensoreinheiten an jeweils unterschiedlichen Positionen der Bioreaktoranlage vorsieht, sodass die Schaumbildung an mehreren Positionen der Bioreaktoranlage überwacht werden kann. Besonders bevorzugt ist dabei eine Kombination aus mindestens einer Patch-Sensoreinheit und mindestens einer Bogen-Sensoreinheit, wobei die mindestens eine Patch-Sensoreinheit insbesondere zur Überwachung eines Schaumniveaus angeordnet ist und die mindestens eine Bogen-Sensoreinheit zur Überwachung des Vorhandenseins von Schaum an einem Port und/oder einem Schlauch und/oder einem Rohr angeordnet ist.

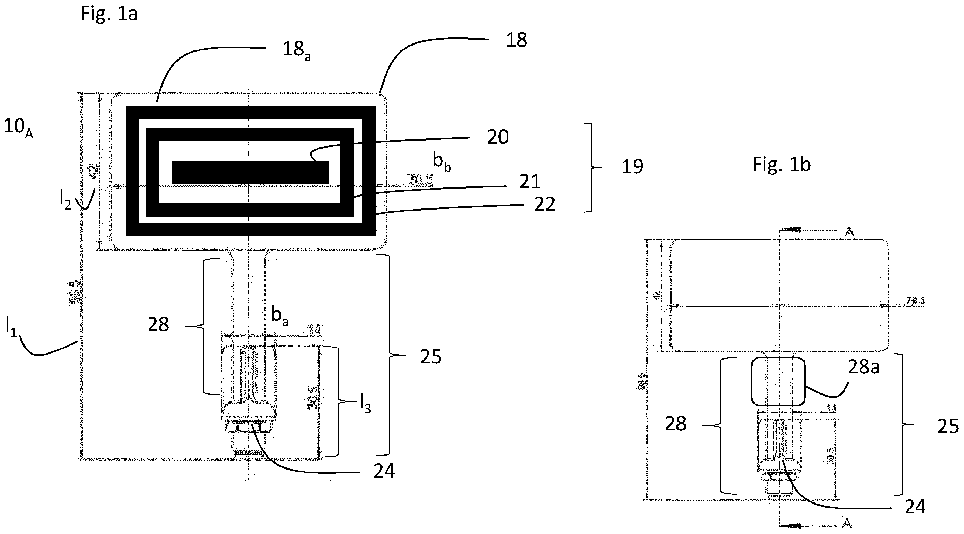

In **Fig. 1a** ist eine beispielhafte kapazitive Patch-Sensoreinheit 10_{A} schematisch dargestellt. Die kapazitive Patch-Sensoreinheit 10_{A} weist ein rechteckiges Feld 18ₐ mit abgerundeten Kanten auf. Das rechteckige Feld 18ₐ kann zumindest teilweise mit einem adhäsiven Klebestreifen 18 zur Anbringung an einer Behälterwandung ausgestattet sein oder ein solches umfassen. Zumindest teilweise an oder auf oder in dem rechteckigen Feld 18ₐ ist ein Elektrodensystem 19 angeordnet. Das Elektrodensystem 19 ist insbesondere als Drei-Elektrodensystem ausgestaltet und weist eine Sensorelektrode 20, eine Schutzelektrode 21 und eine Masseelektrode 22 auf. Alternativ kann das Elektrodensystem 19 im Allgemeinen, also in allen Ausführungsformen, insbesondere in der kapazitiven Patch-Sensoreinheit 10_{A} anstatt als Drei-Elektrodensystem auch als Zwei-Elektrodensystem ausgestaltet sein und lediglich eine Sensorelektrode 20 und eine Schutzelektrode 21 aufweisen. Ferner weist die kapazitive Patch-Sensoreinheit 10_{A} einen Anschlussabschnitt 25 mit einer Anschlusseinheit 24 und einer Konnektierungslippe 28 auf.

Die Konnektierungslippe 28 stellt einen im Wesentlichen länglichen verbindenden Abschnitt dar, insbesondere eine elektrische Leiterbahn zur Versorgung des Elektrodensystems 19 mit Strom bzw. Spannung und/oder zur Leitung von Signalen, an deren einen Ende sich das rechteckige Feld 18ₐ befindet und an dessen anderen Ende sich die Anschlusseinheit 24 befindet. Die Anschlusseinheit 24 dient dabei beispielsweise der Signalübertragung und/oder der Spannungsversorgung. Beispielsweise kann derart durch eine angelegte Wechselspannung ein elektrisches Wechselfeld in dem Elektrodensystem 19 erzeugt und der resultierende Stromfluss als Signal detektiert werden.

Im Wesentlichen funktioniert eine kapazitive Sensoreinheit wie ein offener Kondensator. Insbesondere wird zwischen der Sensorelektrode und der Masseelektrode ein elektrisches Feld aufgebaut. Wenn ein Material mit einer Dielektrizitätszahl *εᵣ* grösser als Luft in das elektrische Feld eindringt, so vergrößert sich je nach *εᵣ* dieses Materials die Kapazität der Sensoranordnung .

Die Sensoreinheit weist ferner eine Elektronikeinheit auf, die diese Kapazitätserhöhung erfassen kann, das Signal, das derart erfasst wird, kann in der nachfolgenden Signalaufbereitung ggf. ausgewertet werden.

Sensoreinheiten, die eine Masseelektrode aufweisen, also auf einem Drei-Elektrodensystem basieren, können ggf. mit der aktiven Fläche, also der Fläche, aus der das elektrische Feld zur Messung im Wesentlichen heraustritt, bündig in ein Material eingebaut und/oder daran angeordnet werden. Da sich bei diesen Sensoreinheiten das elektrische Feld zur Messung von der Sensorelektrode zur integrierten Masseelektrode ausbreitet, entsteht ein definiertes elektrisches Feld zur Messung bzw. ein Messfeld. Solche Sensoreinheiten eignen sich besonders zur Detektion bzw. Erfassung von nicht leitenden Materialien wie z.B. Ölen, Glas, Holz und/oder Kunststoffen. Es können aber ebenso leitende Medien detektiert werden. Ggf. kann eine weitere Kompensationselektrode in die Sensoreinheit verarbeitet werden, insbesondere um die Sensoreinheit unempfindlich gegen mögliche Schmutzablagerungen und Feuchtigkeit auf der Sensorfläche zu machen.

Sensoreinheiten, die keine Masseelektrode aufweisen, also auf einem Zwei-Elektrodensystem basieren, werden in der Regel nicht bündig in ein Material eingebaut und/oder daran angeordnet. Die Masseelektrode ist in diesem Fall nicht in die Sensoreinheit integriert, sondern wird vom zu detektierenden Objekt und/oder Medium, insbesondere von dem Schaum, der gegebenenfalls in der Nähe der Sensoreinheit auftritt, dargestellt. Sensoreinheiten ohne Masseelektrode sind in der Regel relativ unempfindlich gegen Verschmutzung und eignen sich besonders zur Erfassung von Füllständen. Sensoreinheiten, die keine Masseelektrode aufweisen, eignen sich besonders zur Erfassung leitender Medien, insbesondere Medien, die geerdet sind.

Kapazitive Sensoreinheiten können insbesondere sowohl leitende als auch nicht leitende Medien erfassen, die eine Dielektrizitätszahl *εᵣ* > 1 aufweisen. Die Dielektrizitätszahl *εᵣ* (auch Permittivitätszahl oder dielektrische Leitfähigkeit) eines Materials gibt an, um wieviel sich die elektrische Flussdichte erhöht, wenn statt Vakuum oder Luft das entsprechende Material in das Messfeld eindringt.

Leitende Medien weisen insbesondere eine elektrische Leitfähigkeit von > etwa 20 µS/cm auf. Sie können in der Regel von allen Sensortypen, ob mit Masseelektrode oder ohne Masseelektrode, relativ zuverlässig erfasst werden.

Typische Medien, die als leitend erachtet werden, können beispielsweise folgende sein: Wasser mit Ionen bzw. Salz, Blut, Tinte, Milch, Aceton und metallische Stoffe.

Nicht leitende Medien weisen typischerweise eine elektrische Leitfähigkeit von < etwa 20 µS/cm auf. Solche Medien können besonders gut mittels Sensoreinheiten, die eine Masseelektrode aufweisen, also mit Drei-Elektrodensystemen erfasst werden. Wird ein nicht leitendes Objekt in das Feld des Sensors gebracht, verstärkt sich das Feld in Abhängigkeit der Dielektrizitätszahl und der Größe des zu erfassenden Materials und vergrößert damit die Kapazität der Sensoranordnung. Je geringer der Wert *εᵣ* ist, desto schwieriger ist das Medium zu erfassen.

Die Dimensionierung der kapazitiven Patch-Sensoreinheit 10_{A} kann im Allgemeinen beliebig gestaltet werden. Insbesondere kann eine Gesamtlänge I₁ der kapazitiven Patch-Sensoreinheit 10_{A} inklusive dem Anschlussabschnitt 25 und die Anschlusseinheit 24 so gewählt werden, dass sie zwischen etwa 1 cm und etwa 20 cm, insbesondere zwischen etwa 3 cm und etwa 15 cm und bevorzugt zwischen etwa 8 cm und etwa 12 cm liegt. Im beispielhaften vorliegenden Fall beträgt die Gesamtlänge etwa 98,5 mm, also etwa 9,85 cm. Insbesondere kann eine Länge I₂ des rechteckigen Feldes 18ₐ so gewählt werden, dass sie zwischen etwa 0,5 cm und etwa 10 cm, insbesondere zwischen etwa 1 cm und etwa 6 cm und bevorzugt zwischen etwa 3 cm und etwa 5 cm liegt. Im beispielhaften vorliegenden Fall beträgt die Länge I₂ des rechteckigen Feldes 18ₐ etwa 42 mm, also 4,2 cm.

Insbesondere kann eine Breite b_{b} des rechteckigen Feldes 18ₐ so gewählt werden, dass sie zwischen etwa 1 cm und etwa 15 cm, insbesondere zwischen etwa 3 cm und etwa 12 cm und bevorzugt zwischen etwa 5 cm und etwa 10 cm liegt. Im beispielhaften vorliegenden Fall beträgt die Breite b_{b} des rechteckigen Feldes 18ₐ etwa 70,5 mm, also etwa 7,5 cm.

Insbesondere kann eine Breite bₐ der Konnektierungslippe 28 des Anschlussabschnitts 25 so gewählt werden, dass sie zwischen etwa 0,2 cm und etwa 5 cm, insbesondere zwischen etwa 0,5 cm und etwa 3 cm und bevorzugt zwischen etwa 1 cm und etwa 2 cm liegt. Im beispielhaften vorliegenden Fall beträgt die Breite bₐ der Konnektierungslippe 28 des Anschlussabschnitts 25 etwa 14 mm, also etwa 1,4 cm.

Insbesondere kann eine Länge I₃ der Anschlusseinheit 24 so gewählt werden, dass sie zwischen etwa 0,2 cm und etwa 7 cm, insbesondere zwischen etwa 0,5 cm und etwa 5 cm und bevorzugt zwischen etwa 2 cm und etwa 4 cm liegt. Im beispielhaften vorliegenden Fall beträgt die Länge I₃ des Anschlussabschnitts 25 etwa 30,5 mm, also etwa 3,05 cm.

Die beispielhafte kapazitive Patch-Sensoreinheit 10_{A} ist dazu ausgelegt, insbesondere mittels adhäsivem Klebestreifen 18 an einer Behälterhüllen-Innenseite und/oder einer Behälterhüllen-Außenseite und/oder einem anderen Element einer Bioreaktoranlage angeordnet zu werden, sodass das Elektrodensystem 19 bevorzugt kontaktfrei mittels einer kapazitiven Messung eine Änderung der Permittivität erfassen kann und daraus ermittelt werden kann, ob ein Schaum in unmittelbarer Nähe bzw. in unmittelbarer Entfernung bzw. im unmittelbaren Umfeld bzw. Umgebung vorliegt.

Die Anschlusseinheit 24 kann insbesondere einen M8-Stecker aufweisen. Konnektierungslippe 28 des Anschlussabschnitts 25 kann insbesondere mit diesem M8-Stecker verbunden sein und eine Vergussmasse aufweisen, die beide Elemente zusammenhält. Die Vergussmasse kann insbesondere einen ABS Kunststoff aufweisen. Insbesondere ist das Elektrodensystem 19 mittels einer Verteilungsleitung mit der Anschlusseinheit 24 verbunden.

**Fig. 1b** ist eine schematische Darstellung einer beispielhaften Patch-Sensoreinheit 10_{A} mit einem Abstandshalter 28a an der Konnektierungslippe 28. Die Konnektierungslippe 28 dient zum Anschluss des Patches bzw. des rechteckigen Feldes 24, das an einer Oberfläche der Bioreaktoranlage angeordnet werden kann, an die Anschlusseinheit 24 und/oder an ein Transmitterkabel. Dabei bleibt die Konnektierungslippe 28 flexibel, wird also insbesondere nicht an der Oberfläche der Bioreaktoranlage angeordnet, insbesondere fixiert und/oder angeklebt. Ferner erlaubt die Konnektierungslippe 28, den Konnektor bzw. die Anschlusseinheit 24 einfach anbringen und/oder entfernen zu können. Darüber hinaus kann die Konnektierungslippe 28 auch dazu dienen, die spezifizierte Grundkapazität der Patch-Sensoreinheit 10_{A} in einen gewünschten Bereich zu bringen, indem sie die Leiterbahnen der Patch-Sensoreinheit verlängert.

Die Konnektierungslippe 28 kann ggf. sensibel sein, das heißt mit anderen Worten dazu beitragen, dass eine Änderung in der Permittivität oder Kapazität erzeugt und/oder gemessen wird. Insbesondere kann eine Permittivitätsänderung hinter der Lippe zur gemessenen Kapazität und somit zum Gesamtsignal des Sensors beitragen.

Da die Konnektierungslippe 28 ggf. keine im Wesentlichen definierte Position aufweist weil sie bevorzugt nicht angeklebt wird an der Oberfläche der Bioreaktoranlage können falsch-positive Signale erzeugt werden, wenn die Konnektierungslippe 28 im Prozessverlauf bewegt wird (z.B. durch Zug am Kabel oder Druckvariation im Bioreaktor). Ein Aufkleben der Konnektierungslippe 28 stellt allerdings keine bevorzugte Ausführungsform dar, da die Konnektierungslippe 28 dann nicht mehr flexibel bzw. beweglich wäre und sich die Konnektierungslippe 28 durch Kabelbewegung auch wieder leicht ablösen würde.

Ein Abstandshalter 28a der sensiblen Fläche, welche zumindest teilweise die Konnektierungslippe 28 umschließt und/oder bedeckt kann dazu dienen, solche unerwünschten Effekte, wie das Messen eines falsch-positiven Signals zu verringern oder gar zu vermeiden. Der Abstandshalter 28a kann insbesondere einen Mantel darstellen, der beispielsweise aus Polypropylen (PP) besteht oder aufweist. Alternative abschirmende Stoffe mit niedrigem*εᵣ*, insbesondere Kunststoffe oder Schaumstoffe können auch in Frage kommen.

Der Abstandshalter 28a kann insbesondere eine Dicke von etwa 0,2 mm bis etwa 3 mm, bevorzugt von etwa 0,8 mm bis etwa 1,3 mm und besonders bevorzugt von etwa 1 mm aufweisen. Eine Dicke von etwa 1 mm erlaubt eine hohe Flexibilität der Konnektierungslippe 28 und sichert gleichzeitig einen ausreichenden Abstand von möglichen Fremdkörpern, um eine Signaleinkopplung in den sensitiven Bereichen der Konnektierungslippe zu verhindern.

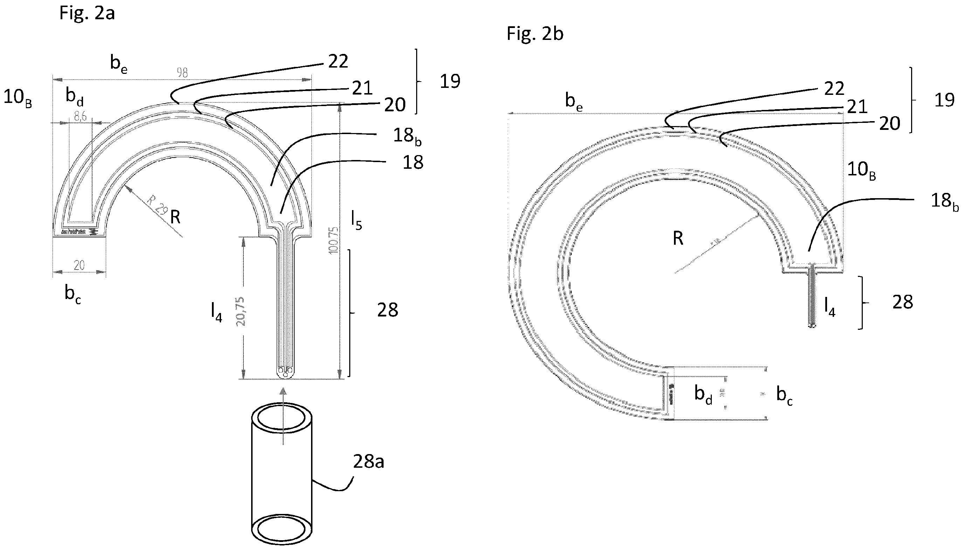

In **Fig. 2a** und **2b** sind zwei beispielhafte kapazitive Bogen-Sensoreinheiten 10_{B} schematisch dargestellt. Die Merkmale, die für die kapazitive Patch-Sensoreinheit 10_{A} genannt wurden, können auch in den kapazitiven Bogen-Sensoreinheiten 10_{B} Anwendung finden, sofern sie sich damit kombinieren lassen. In **Fig. 2a** ist eine beispielhafte kapazitive Bogen-Sensoreinheit 10_{B} dargestellt, die einen Bogenabschnitt 18_{b} aufweist, der im Wesentlichen einem Halbkreisbogen entspricht.

Der Bogenabschnitt 18_{b} dient im vorliegenden Fall insbesondere der Anordnung eines Elektrodensystems 19. Die Funktion des Bogenabschnitts 18_{b} entspricht zumindest teilweise der Funktion des rechteckigen Feldes 18ₐ in einer Patch-Sensoreinheit 10_{A}. Auch der Bogenabschnitt 18_{b} kann einen adhäsiven Klebestreifen 18 oder ein adhäsives Klebefeld aufweisen.

Die kapazitive Bogen-Sensoreinheit 10_{B} weist ein Elektrodensystem 19 auf, das auf dem Bogenabschnitt 18_{b} und auf der Konnektierungslippe 28 eines Sensorabschnitts 25 angeordnet ist. Das Elektrodensystem 19 umfasst eine Sensorelektrode 20, eine Schutzelektrode 21 und bevorzugt eine Masseelektrode 22. Die Elektroden 20, 21, 22 ahmen zumindest teilweise die Form der kapazitiven Bogen-Sensoreinheit 10_{B}, insbesondere der Bogenform des Bogenabschnitts 18_{b} nach, indem sie jeweils entlang der Kanten der kapazitiven Bogen-Sensoreinheit 10_{B} angeordnet sind und jeweils einen geschlossenen Kreislauf bilden.

Alternativ kann das Elektrodensystem 19 im Allgemeinen, also in allen Ausführungsformen, insbesondere in der kapazitiven Bogen-Sensoreinheit 10_{B} anstatt als Drei-Elektrodensystem auch als Zwei-Elektrodensystem ausgestaltet sein und lediglich eine Sensorelektrode 20 und eine Schutzelektrode 21 und keine Masseelektrode 22 aufweisen.

Ein Abstandshalter 28a ist schematisch dargestellt. Der Abstandshalter 28a kann zumindest teilweise über die Konnektierungslippe 28 angeordnet werden, sodass sie diese zumindest teilweise umschließt.

Die Dimensionierung der kapazitiven Bogen-Sensoreinheit 10_{B} kann im Allgemeinen beliebig gestaltet werden. Insbesondere kann eine Gesamtlänge l₅ der kapazitiven Bogen-Sensoreinheit 10_{B} inklusive der Konnektierungslippe 28 des Sensorabschnitts 25 so gewählt werden, dass sie zwischen etwa 1 cm und etwa 20 cm, insbesondere zwischen etwa 5 cm und etwa 15 cm und bevorzugt zwischen etwa 8 cm und etwa 12 cm liegt. Im beispielhaften vorliegenden Fall beträgt die Gesamtlänge l₅ etwa 100,75 mm, also etwa 10 cm.

Insbesondere kann eine Gesamtbreite bₑ der kapazitiven Bogen-Sensoreinheit 10_{B} zwischen den beiden äußeren Kanten der kapazitiven Bogen-Sensoreinheit 10_{B} so gewählt werden, dass sie zwischen etwa 1 cm und etwa 15 cm, insbesondere zwischen etwa 5 cm und etwa 13 cm und bevorzugt zwischen etwa 8 cm und etwa 12 cm liegt. Im beispielhaften vorliegenden Fall beträgt die Gesamtbreite bₑ etwa 98 mm, also 9,8 cm.

Insbesondere kann eine Breite b_{c} des bogenförmigen Streifens der kapazitiven Bogen-Sensoreinheit 10_{B} zwischen den beiden äußeren Kanten des Streifens so gewählt werden, dass sie zwischen etwa 0,2 cm und etwa 5 cm, insbesondere zwischen etwa 1 cm und etwa 4 cm und bevorzugt zwischen etwa 1,5 cm und etwa 3 cm liegt. Im beispielhaften vorliegenden Fall beträgt die Breite b_{c} des bogenförmigen Streifens der kapazitiven Bogen-Sensoreinheit 10_{B} etwa 20 mm, also 2 cm.

Insbesondere kann eine Mindestbreite b_{d} des Bogens, die die Elektroden nachbilden so gewählt werden, dass sie zwischen etwa 0,1 cm und etwa 1,5 cm, insbesondere zwischen etwa 0,4 cm und etwa 1,2 cm und bevorzugt zwischen etwa 0,6 cm und etwa 1,0 cm liegt. Im beispielhaften vorliegenden Fall beträgt die Mindestbreite b_{d} des Bogens etwa 8,6 mm, also 0,86 cm.

Insbesondere kann eine Länge l₄ der Konnektierungslippe 28 des Sensorabschnitts 25, so gewählt werden, dass sie zwischen etwa 0,1 cm und etwa 3,5 cm, insbesondere zwischen etwa 1 cm und etwa 2,5 cm und bevorzugt zwischen etwa 1,5 cm und etwa 2,3 cm liegt. Im beispielhaften vorliegenden Fall beträgt die Länge l₄ der Konnektierungslippe 28 des Sensorabschnitts 25 etwa 20,75 mm, also etwa 2,1 cm.

Vorliegend weist die kapazitive Bogen-Sensoreinheit 10_{B} einen Bogenabschnitt 18_{b} mit einem einheitlichen Radius R auf, der zwischen etwa 0,8 cm und etwa 10 cm, insbesondere zwischen etwa 1,3 cm und etwa 5 cm und bevorzugt zwischen etwa 1,8 cm und etwa 2,4 cm liegen kann. Im gezeigten Beispiel beträgt der Radius R etwa 29 mm, also etwa 2,9 cm. Der Bogenabschnitt 18_{b} kann alternativ auch einen sich ändernden Radius aufweisen, was beispielsweise bei einer Ellipsenfom der Fall wäre.

Im Allgemeinen weist der Bogen eines Bogenabschnitts 18_{b} eine gekrümmte Form auf. Die gekrümmte Form kann durch mindestens einen Krümmungsradius r beschrieben werden. Der Bogen kann im Wesentlichen die Form mindestens eines Abschnitts eines Kreisbogens aufweisen, beispielsweise etwa eines Viertel-, Drittel-, Halb-, Zweidrittel-, oder eines Dreiviertel-Kreisbogens oder etwa eines ganzen Kreisbogens. Alternativ kann der Bogen zumindest im Wesentlichen einem Abschnitt eines von einem Kreisbogen abweichenden Bogens, beispielsweise eines Ellipsenbogens entsprechen. Auch andere Bogenformen mit unregelmäßigem Radius r sind denkbar.

In **Fig. 2b** ist eine beispielhafte kapazitive Bogen-Sensoreinheit 10_{B} dargestellt, die einen Bogenabschnitt 18_{b} aufweist, der im Wesentlichen mehr als 180° eines Kreisbogens aufweist, insbesondere im Wesentlichen einem dreiviertel Kreisbogen entspricht. In anderen Worten entspricht der Bogenabschnitt 18_{b} in etwa 270° eines Kreisbogens. Die Dimensionierung, die bereits für die beispielhafte kapazitive Bogen-Sensoreinheit 10_{B} in **Fig. 2a** genannt ist, kann in entsprechender Weise auch für die vorliegende beispielhafte kapazitive Bogen-Sensoreinheit 10_{B} der **Fig. 2a** angewandt werden.

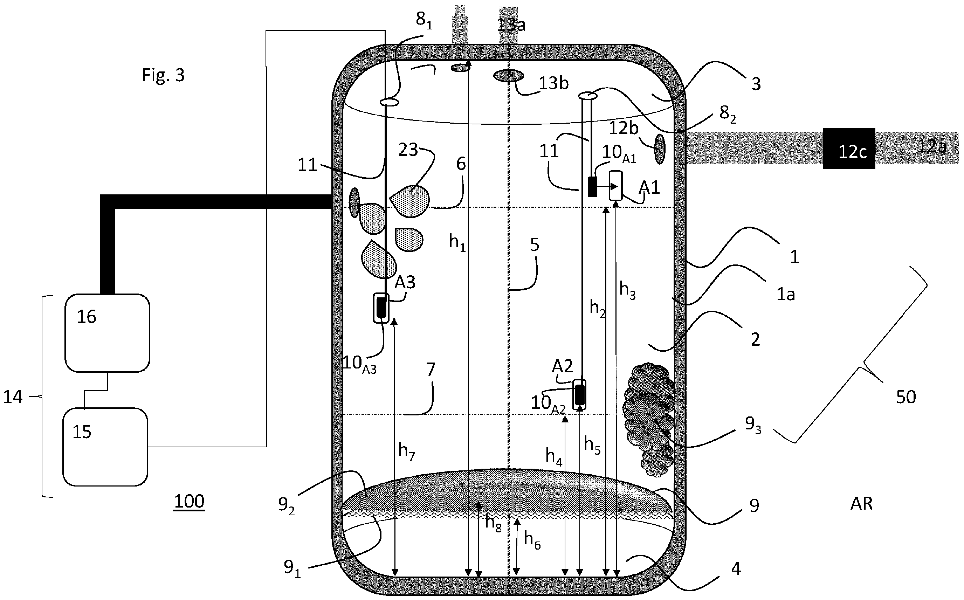

**Fig. 3** ist eine schematische Darstellung eines Systems 100 zum Erfassen von Schaum bzw. schaumigem Medium 9₂, 9₃. Insbesondere ist eine beispielhafte Bioreaktoranlage 50 dargestellt, die schematisch an- bzw. aufgeschnitten ist, um einen Einblick in den Innenraum des Einwegbehälters 1 zu geben. Die Behälterhülle kann insbesondere eine Kunststoff-Folie sein oder aufweisen. Die Kunststoff-Folie kann insbesondere mindestens eines der folgenden Materialien aufweisen: Polyolefine, insbesondere Polyethylen (PE) oder Polypropylen (PP), Polyvinylchlorid (PVC), Polystyrol (PS), verschiedene Polyester sowie Polycarbonat (PC) und Polyethylenterephthalat (PET).

In dem Behälter-Innenraum 2 steht über dem Behälter-Innenboden 4 ein Medium bzw. eine Bioreaktor-Medienfüllung 9. Die Bioreaktor-Medienfüllung 9 kann ein flüssiges Medium 9₁ aufweisen, auf dem sich ein Schaum 9₂ gebildet hat. Zusätzlich oder alternativ kann an der Behälterhüllen-Innenseite 1a auch ein Schaum 9₃ vorhanden sein, der sich entweder dort gebildet hat und/oder bei der Füllstandänderung an der Behälterhüllen-Innenseite 1a haften blieb. Des Weiteren kann sich ein Schaum 9₃ insbesondere auch in und/oder an einem Abluftschlauch 12a und/oder an einem anderen Rohr oder Schlauch bilden. Beispielsweise kann auch ein Schaum in ein Rohr 13a dringen, das als Ober-Zugang dienen kann und an einem entsprechenden Port 13b der Behälter-Innendecke 3 angeordnet ist.

Um die Anwesenheit von Schaum 9₂, 9₃ erfassen zu können, sind zumindest zwei, insbesondere drei kapazitive Patch-Sensoreinheiten 10_{A1}, 10_{A2}, 10_{A3} auf der Behälterhüllen-Innenseite 1a an jeweiligen unterschiedlichen Anordnungspositionen, insbesondere an den Anordnungspositionen A1, A2, A3 entsprechend angeordnet. Eine erste kapazitive Patch-Sensoreinheit 10_{A1} wird gerade in der Nähe knapp oberhalb einer Grenzlinie 6, die den maximalen Füllstand anzeigt, angeordnet. Der Pfeil signalisiert die Richtung, in der die erste kapazitive Patch-Sensoreinheit 10_{A1} an der Anordnungspositionen A1 angeordnet wird. Eine zweite kapazitive Patch-Sensoreinheit 10_{A2} ist in der Nähe knapp oberhalb einer Grenzlinie 7, die den minimalen Füllstand anzeigt bzw. entspricht, angeordnet. Eine dritte kapazitive Patch-Sensoreinheit 10_{A3} ist zwischen den Grenzlinien 7, 8 angeordnet.

Mit dem Begriff "knapp oberhalb" ist eine Distanz zwischen der jeweiligen genannten Höhe und der Unterkante der Sensoreinheit gemeint, die zwischen etwa 0,2 cm und etwa 10 cm, insbesondere zwischen 0,5 cm und 5 cm und bevorzugt zwischen etwa 1 cm und 3 cm liegt.

Zumindest ein Teil der kapazitiven Patch-Sensoreinheiten 10_{A1}, 10_{A2}, 10_{A3} kann alternativ auch zur berührungsfreien Messung auf der Behälterhüllen-Außenseite 1b angeordnet sein. Es können insbesondere auch zusätzliche Sensoreinheiten zur Erfassung des Schaumniveaus an der Bioreaktoranlage 50 angeordnet sein. Die Anordnung der kapazitiven Patch-Sensoreinheiten 10_{A1}, 10_{A2}, 10_{A3} eignet sich insbesondere dazu, dass ermittelt werden kann, ob sich ein Medium 9, insbesondere ein Schaum 9₂, 9₃ auf einem tolerierbaren Niveau zwischen dem minimalen und dem maximalen Füllstand befindet, oder ob ggf. insbesondere Schaum 9₂, 9₃ über die Grenzlinie 6, die den maximalen Füllstand anzeigt, hinauswächst und möglicherweise in eines der Rohre 12a, 13a treten kann.

Die Grenzlinie 7, die den minimalen Füllstand anzeigt, ist dazu geeignet anzuzeigen, ob der Einwegbehälter 1 ausreichend mit einem Medium 9, insbesondere einem fluiden Medium 9₁ befüllt ist, was insbesondere der Fall wäre, wenn die Höhe h₆ des fluiden Mediums 9₁ in etwa auf der Höhe der Grenzlinie 7 läge. Die Grenzlinie 6, die den maximalen Füllstand anzeigt bzw. entspricht, ist dazu geeignet anzuzeigen, ob der Einwegbehälter 1 maximal mit einem Medium 9, insbesondere einem fluiden Medium 9₁ befüllt ist, was insbesondere der Fall wäre, wenn die Höhe h₆ des fluiden Mediums 9₁ oder die Höhe h₈ des darüber stehenden Schaums in etwa auf der Höhe der Grenzlinie 6 läge. Eine Höhe entspricht dabei einem Abstand von einem Behälter-Innenboden 4 zum maximal höchsten Punkt des Schaums 9₂, 9₃ oder des fluiden Mediums 9₁.

Der Abstand von dem Behälter-Innenboden 4 zum Grenzlinie 7 entspricht der Höhe h₄ und der Abstand von dem Behälter-Innenboden 4 zum Grenzlinie 6 entspricht der Höhe h₂. Der Abstand von dem Behälter-Innenboden 4 zur Unterkante der ersten Patch-Sensoreinheit 10_{A1} entspricht der Höhe h₃. Der Abstand von dem Behälter-Innenboden 4 zur Unterkante der zweiten Patch-Sensoreinheit 10_{A2} entspricht der Höhe h₅. Der Abstand von dem Behälter-Innenboden 4 zur Unterkante der dritten Patch-Sensoreinheit 10_{A3} entspricht der Höhe h₇. Der Abstand von dem Behälter-Innenboden 4 zur Behälter-Innendecke 3 entspricht der Höhe h₁.

Bevorzugt ist eine der kapazitiven Sensoreinheiten 10_{A1}, 10_{A2}, 10_{A3} dazu ausgelegt, abgeschaltet bzw. inaktiviert zu werden. Insbesondere ist eine kapazitive Sensoreinheit 10_{A2}, die an einer Anordnungsposition A2 im unteren Drittel der Bioreaktoranlage 50 angeordnet ist, dazu ausgelegt abgeschaltet zu werden. Es kann eine Patch- 10_{A1}, 10_{A2}, 10_{A3} und/oder eine Bogen-Sensoreinheit 10_{B} abgeschaltet werden.

Optional ist das System 100 dazu ausgelegt, eine der kapazitiven Sensoreinheiten 10_{A1}, 10_{A2}, 10_{A3} abzuschalten. Insbesondere ist das System 100 dazu ausgelegt, eine kapazitive Sensoreinheit 10_{A2}, die an einer Anordnungsposition A2 im unteren Drittel der Bioreaktoranlage 50 angeordnet ist, abzuschalten.

Es können auch mehrere kapazitiven Sensoreinheiten 10_{A1}, 10_{A2}, 10_{A3} abgeschaltet werden, je nachdem auf welches Niveau die Bioreaktoranlage 50 befüllt wird bzw. welchen Füllstand das Medium innerhalb der Bioreaktoranlage 50 aufweist. Insbesondere bei Fed-Batch Prozessen können unterschiedliche Füllstände zu unterschiedlichen Prozessen innerhalb der Bioreaktoranlage 50 erreicht werden. Dies hätte zur Folge, dass eine oder mehrere kapazitive Sensoreinheiten 10_{A1}, 10_{A2}, 10_{A3} anstatt der Anwesenheit oder nicht-Anwesenheit eines Schaums die Anwesenheit eines Mediums erfassen, was zu einer Fehlinterpretation der Datenerfassung führen kann. Beispielsweise kann somit fälschlicherweise angenommen werden, dass ein Schaum vorliegt im unmittelbaren Umfeld einer kapazitiven Sensoreinheit 10_{A1}, 10_{A2}, 10_{A3}, da diese eine Änderung in der Permittivität erfasst, die durch die das Befüllen mit dem Medium hervorgerufen wird. Zu diesem Zweck kann die Bioreaktoranlage 50, das System 100 oder die kapazitiven Sensoreinheit 10_{A1}, 10_{A2}, 10_{A3} dazu ausgelegt sein die jeweilige kapazitive Sensoreinheit 10_{A1}, 10_{A2}, 10_{A3} automatisch und/oder manuell abzuschalten bzw. inaktiv zu stellen. Dabei bedeutet "abgeschaltet", dass ggf. kein Zustand erfasst wird zumindest jedoch keine Daten von der jeweiligen abgeschalteten kapazitiven Sensoreinheit 10_{A1}, 10_{A2}, 10_{A3} weitergeleitet werden.

Die kapazitiven Patch-Sensoreinheiten 10_{A1}, 10_{A2}, 10_{A3} sind mittels Kabel 11 mit einer Kontrolleinheit 14 verbunden, um beispielsweise Daten übertragen zu können, gesteuert zu werden und/oder mit Strom versorgt zu werden. Alternativ kann die Datenübertragung auch kabellos erfolgen. Die Kabel 11 werden dabei durch jeweils ein Kabelport 8₁, 8₂ von dem Behälter-Innenraum 2 in den Behälter-Außenraum AR geführt.

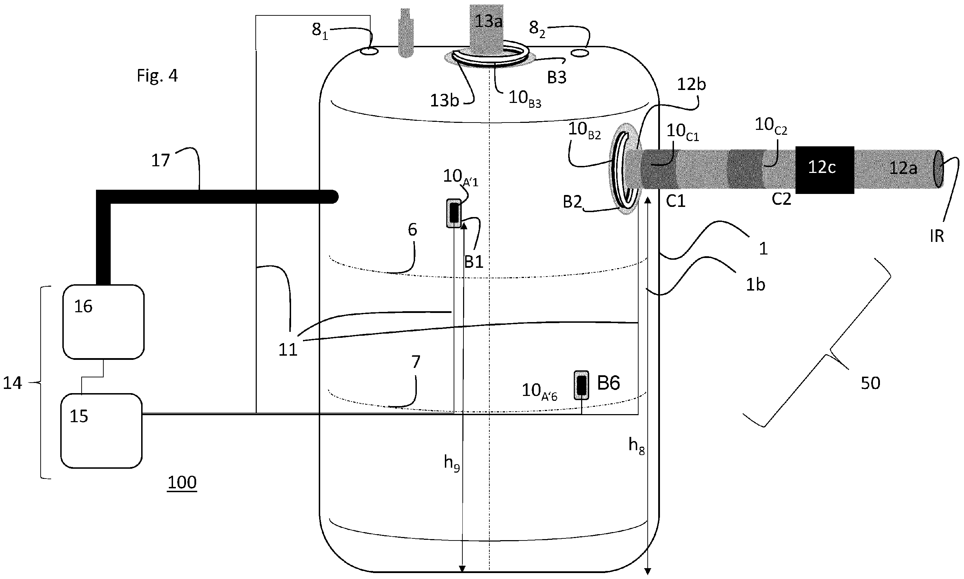

**Fig. 4** ist eine schematische Darstellung der beispielhaften Bioreaktoranlage 50 aus **Fig. 3** mit Ansicht von außen. Zwei kapazitive Bogen-Sensoreinheiten 10_{B2}, 10_{B3} sind von dem Außenraum AR der Bioreaktoranlage 50 an der Behälterhüllen-Außenseite 1b an den Anordnungspositionen B2, B3 angeordnet. Weitere Patch-Sensoreinheiten 10_{A'1}, 10_{A'6} sind ebenfalls an der Behälterhüllen-Außenseite 1b an den Anordnungspositionen B1, B6 angeordnet. Beispielsweise ist die Patch-Sensoreinheit 10_{A'1} an der Anordnungsposition B1 auf der Höhe h₉ oberhalb der Grenzlinie 6 und unterhalb des Ports 12b des Abluftschlauchs 12a angeordnet, um das Vorliegen eines hohen Schaumniveaus erfassen zu können, welches möglicherweise ohne Treffen einer Gegenmaßnahme zur Schaumbildung in den Abluftschlauch 12b treten kann.

Die Patch-Sensoreinheiten 10_{A'1}, 10_{A'6} sind dazu ausgelegt, berührungsfrei und/oder durch die Behälterwand hindurch ein Schaumniveau innerhalb der Bioreaktoranlage 50, insbesondere innerhalb der Einwegbehälters 1 erfassen zu können. Sofern Patch-Sensoreinheiten 10_{A1}, 10_{A2}, 10_{A3} im Behälter-Innenraum 2 angeordnet sind, können die Patch-Sensoreinheiten 10_{A'1}, 10_{A'6} auf der Behälterhüllen-Außenseite 1b optional sein. Andernfalls können zwei oder mehr Patch-Sensoreinheiten an der Behälterhüllen-Außenseite 1b eine Alternative zu den Patch-Sensoreinheiten 10_{A1}, 10_{A2}, 10_{A3} auf der Behälterhüllen-Außenseite 1b darstellen.

Die kapazitive Bogen-Sensoreinheiten 10_{B2}, 10_{B3} sind dazu ausgelegt, das Vorhandensein von Schaum 9₂, 9₃ an kritischen Positionen B2, B3 zu erfassen. Beispielsweise ist eine Bogen-Sensoreinheit 10_{B2} an einem Port 12b des Abluftschlauchs 12a an der Anordnungsposition B2 angeordnet, um derart erfassen zu können, ob Schaum 9₂, 9₃ in den Abluftschlauch 12a tritt. Derart kann beispielsweise verhindert werden, dass Schaum 9₂, 9₃ einen Filter 12c an dem Abluftschlauch 12a verstopft und/oder kontaminiert.

Mindestens eine, insbesondere zwei (weitere) Sensoreinheiten, und zwar zwei Manschetten-Sensoreinheiten 10_{C1}, 10_{C2} sind an der Außenseite an dem Abluftschlauch 12a jeweils an einer oder mehreren Anordnungspositionen C1 und C2 angeordnet. Die Manschetten-Sensoreinheit 10_{C1}/10_{C2} ist bevorzugt ähnlich zu der Patch-Sensoreinheit 10_{A} und/oder der Bogen-Sensoreinheit 10_{B} eine kapazitive Sensoreinheit.

Die Manschetten-Sensoreinheit 10_{C1} ist insbesondere direkt bzw. unmittelbar hinter dem Port 12b an der Anordnungsposition C1 an dem Abluftschlauch 12a angeordnet. Diese Manschetten-Sensoreinheit 10_{C1} kann erfassen, ob bereits Schaum 9₂, 9₃ in den Innenraum IR des Abluftschlauchs 12a getreten ist. Die Manschetten-Sensoreinheit 10_{C1} kann genau wie die Manschetten-Sensoreinheit 10_{C2} ein (bevorzugt im Wesentlichen rechteckiger) Streifen oder ein (bevorzugt im Wesentlichen rechteckiger) Patch sein, der insbesondere entlang seiner Längsachse zumindest teilweise um den Umfang eines Schlauchs und/oder Rohrs bzw. Leitung gelegt wird bzw. werden kann und somit eine Manschette um den Umfang des Rohres bildet. In anderen Worten ist die Form dieser Sensoreinheit bevorzugt wie ein Streifen, der mit seiner Fläche zumindest teilweise um den Schlauch herum aufgeklebt wird. Die Manschetten-Sensoreinheiten 10_{C1} und/oder 10_{C2} bilden insbesondere dann einen Bogen, wenn sie um den Schlauch bzw. das Rohr bzw. die Leitung herum gelegt werden, wobei sie vor dem Herumlegen beispielsweise lediglich eine (bevorzugt im Wesentlichen rechteckige) Sensoreinheit darstellen.

Eine Manschetten-Sensoreinheit 10_{C1} und/oder 10_{C2} kann im Allgemeinen dazu ausgelegt sein, ein Rohr und/oder einen Schlauch mindestens im Wesentlichen zur Hälfte, bevorzugt mindestens im Wesentlichen zu Zweidrittel und besonders bevorzugt im Wesentlichen zu Dreiviertel des Umfangs zu umschließen. Vor dem Anbringen an den Schlauch und/oder das Rohr hat die Manschetten-Sensoreinheit 10_{C1} und/oder 10_{C2} beispielsweise die Form einer Patch-Sensoreinheit oder ähnelt dieser Form zumindest. In anderen Worten weist die Manschetten-Sensoreinheit 10_{C1}/10_{C2} vor dem Anlegen bzw. Anbringen an einem Rohr bzw. einer Leitung im Wesentlichen keine Krümmung auf und hat insbesondere eine im Wesentlichen zweidimensionale ebene Fläche, die eine flache Ebene bildet.

Mit dem zumindest teilweisen Umschließen eines Rohres bzw. einer Leitung nimmt die Manschetten-Sensoreinheit 10_{C1}/10_{C2} eine Biegung ein, wodurch keine flache Ebene sondern eine gekrümmte Fläche entsteht, deren Krümmung sich insbesondere durch den Radius des Rohres bestimmt. Bevorzugt wird die Manschetten-Sensoreinheit mit ihrer Längsachse im Wesentlichen senkrecht zu Längsachse des Rohres bzw. der Leitung um das Rohr bzw. die Leitung herum gelegt. Die Biegung der Manschetten-Sensoreinheit 10_{C1}/10_{C2} ist dabei im Wesentlichen koaxial mit der Längsachse (zumindest eines Teils) des Rohres bzw. der Leitung. Alternativ kann die Manschetten-Sensoreinheit 10_{C1}/10_{C2} auch "schräg" an dem Rohr bzw. der Leitung angebracht werden, was heißt, dass die Längsachse der Manschetten-Sensoreinheit 10_{C1}/10_{C2} nicht senkrecht mit der Längsachse des Rohres bzw. Leitung ist.

Prinzipiell können alle Sensoreinheiten, also auch die Bogen- und die Patch-Sensoreinheit eine Krümmung aufweisen, die im Wesentlichen durch den Radius des Elements bestimmt wird, an dem sie angebracht sind. In der Regel ist dieser Radius allerdings größer als im Fall einer Manschetten-Sensoreinheit 10_{C1}/10_{C2}, da die Manschetten-Sensoreinheit 10_{C1}/10_{C2} i.d.R. an Rohren bzw. Leitungen und nicht am Behälter einer Bioreaktoranlage angebracht wird. Die Krümmung der Manschetten-Sensoreinheit 10_{C1}/10_{C2} ist aus diesem Grund in der Regel stärker als bei der Bogen-Sensoreinheit 10_{B} bzw. der Patch-Sensoreinheit 10_{A}.

Diese Krümmung, die sich daraus ergibt, dass die Sensoreinheiten 10_{A} 10_{B}, 10_{C} flexibel sind und an die (bevorzugt äußere) Form einer Oberfläche z.B. eines Einwegbehälters bzw. Bioreaktors 1 angelegt werden können ist nicht zu verwechseln mit der Krümmung des Bogens einer Bogen-Sensoreinheit 10_{B}. Der Bogen einer Bogen-Sensoreinheit 10_{B} kann in einer zweidimensionalen Ebene liegen, insbesondere dann, wenn die Bogen-Sensoreinheit 10_{B} noch nicht an der Anlage 100 bzw. Behälter 1 angeordnet wurde. Eine Bogen-Sensoreinheit 10_{B} weist in anderen Worten bereits einen Bogen in ihrer zweidimensionalen Form auf, wobei diese in einer Ebene liegt bevor sie an eine Bioreaktoranlage 100 bzw. einem Behälter 1 angeordnet wird. Falls die Fläche an der die Bogen-Sensoreinheit 10_{B} angeordnet wird, einer flachen ungekrümmten Ebenen entspricht, kann die Bogen-Sensoreinheit 10_{B} zumindest teilweise um einen Port (z.B. Port 12b) herum angeordnet werden, ohne dass sie dabei eine Krümmung ihrer Oberfläche erfährt. Die Manschetten-Sensoreinheit 10c weist hingegen immer eine Krümmung auf wenn sie an einem Schlauch bzw. Rohr bzw. einer Leitung angeordnet wird.

Die Distanz zwischen Port 12b und Anordnungsposition C1 liegt beispielsweise zwischen etwa 2 cm und etwa 30 cm, insbesondere zwischen etwa 5 cm und etwa 15 cm und bevorzugt zwischen etwa 7 cm und etwa 12 cm. Die weitere Manschetten-Sensoreinheit 10_{C2} ist direkt vor dem Filter 12c an der Anordnungsposition C2 an dem Abluftschlauch 12a angeordnet. Diese Manschetten-Sensoreinheit 10_{C2} kann erfassen, ob bereits Schaum 9₂, 9₃ in den Innenraum IR des Abluftschlauchs 12a getreten ist, und zwar in unmittelbarer Nähe des Filters 12c. Die Distanz zwischen Filter 12c und Anordnungsposition C2 liegt beispielsweise zwischen etwa 2 cm und etwa 30 cm, insbesondere zwischen etwa 5 cm und etwa 15 cm und bevorzugt zwischen etwa 7 cm und etwa 12 cm.

Der Abluftschlauch 12a ist an einer vergleichsweise hohen Position, beispielsweise in der oberen Hälfte, insbesondere im oberen Drittel und bevorzugt im oberen Viertel des Einwegbehälters 1 angeordnet. Die Distanz zwischen Behälter-Innenboden 4 und Unterkante des Ports 12b für den Abluftschlauch 12a entspricht der Höhe h₈.

Eine weitere Bogen-Sensoreinheit 10_{B3} ist an dem Port 13b für den Ober-Zugang 13a angeordnet, um an dieser Anordnungsposition B3 erfassen zu können, ob ein Schaum in den Schlauch des Ober-Zugangs 13a tritt oder treten wird.

Die kapazitiven Bogen-Sensoreinheiten 10_{B2}, 10_{B3} sind mittels Kabel 11 mit der Kontrolleinheit 14 verbunden. Dabei kann die Recheneinheit 15 der Kontrolleinheit 14 Daten empfangen und ggf. auswerten und/oder verarbeiten und an die Steuer- und/oder Regeleinheit 16 weitergeben, sodass die Steuer- und/oder Regeleinheit 16 eine Maßnahme erfassen kann, insbesondere wenn ein kritischer Zustand erfasst wird. Beispielsweise kann die Steuer- und/oder Regeleinheit 16 veranlassen, dass über einen Schlauch 17 dem Behälter-Innenraum 2 ein Antischaum-Mittel 23 zugefügt wird, sodass eine Schaumbildung reduziert und/oder unterdrückt werden kann. Insbesondere können mehrere Ports an der Bioreaktoranlage 50 angeordnet sein, sodass lokal am Ort des Vorliegens eines Schaums 9₂, 9₃ ein Antischaum-Mittel 23 zum Einsatz kommen kann.

Maßnahmen können zumindest eine der folgenden sein: Auslösen eines sichtbaren und/oder hörbaren Alarms, Auslösen der Zugabe von Antischaum-Mittel 23, Anhalten der Zugabe von Antischaum-Mittel 23, Abschalten einer schaumbildenden Apparatur, beispielsweise Abschalten einer Rührvorrichtung und/oder Abschalten einer Substanzzugabe.

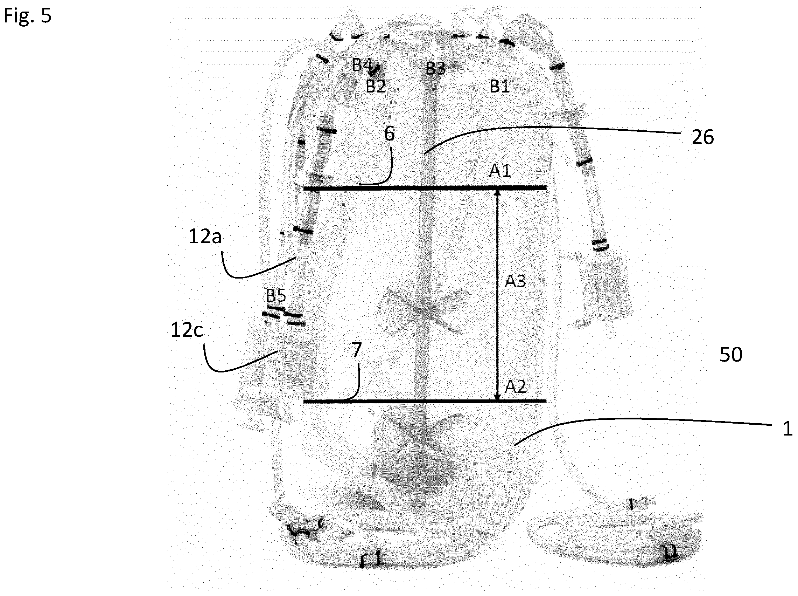

**Fig. 5** ist eine realistische Darstellung einer beispielhaften Bioreaktoranlage 50 mit Ansicht von außen. Insbesondere weist die Bioreaktoranlage 50 einen Einweg-Behälter 1 auf, der einen Einweg-Beutel darstellt. Die Bioreaktoranlage 50 weist darüber hinaus auch eine Rühreinheit 26 zum Rühren bzw. Mischen eines Mediums auf. Neben mehreren Schläuchen weist die Bioreaktoranlage 50 einen Abluftschlauch 12a mit einem Filter 12c auf. In der Darstellung sind ein minimaler Füllstand 7 und ein maximaler Füllstand 6 mittels Markierungsstrichen angedeutet. Ferner sind die Anordnungspositionen A1, A2 und A3, an denen bevorzugt Patch-Sensoreinheiten angeordnet werden können, in der Darstellung angedeutet. An diesen Anordnungspositionen A1, A2 und A3 lässt sich insbesondere das Schaumniveau überwachen. Darüber hinaus sind Anordnungspositionen B1, B2, B3, C1, C2, an denen bevorzugt Bogen-Sensoreinheiten (oder andere Typen von Sensoreinheiten, wie eine Patch-Sensoreinheit 10_{A'1} oder eine Manschetten-Sensoreinheit 10_{C1}, 10_{C2}) angeordnet werden können, in der Darstellung angedeutet. An diesen Anordnungspositionen B1, B2, B3, C1, C2 lässt sich insbesondere ein kritischer Zustand erfassen, wenn beispielsweise Schaum in der Nähe eines Ports, insbesondere in der Nähe des Ports für den Abluftschlauch 12a oder gar in der Nähe des Filters 12c vorhanden ist.

Der maximale Füllstand 6 ist insbesondere ein vorbestimmter Wert für die empfohlene Maximal-Befüllung mit einem Medium, der beispielsweise anhand einer Füllstandsmarkierung an und/oder in der Bioreaktoranlage, insbesondere an und/oder in dem Einwegbehälter angedeutet ist. Der minimale Füllstand 7 ist insbesondere ein vorbestimmter Wert für die empfohlene Minimal-Befüllung mit einem Medium, der beispielsweise anhand einer Füllstandsmarkierung an und/oder in der Bioreaktoranlage, insbesondere an und/oder in dem Einwegbehälter angedeutet ist.

Insbesondere ist der sichere und zuverlässige Betrieb der Bioreaktoranlage empfohlen für die Befüllung mit einem Medium innerhalb der Grenzen zwischen etwa dem maximalen Füllstand 6 und etwa dem minimalen Füllstand 7.

Insbesondere können die jeweiligen Anordnungspositionen an der entsprechenden Bioreaktoranlage eingezeichnet bzw. markiert sein, sodass der Verwender die Sensoreinheit in einfacher Weise an den entsprechenden Anordnungspositionen anordnen kann.

Eine kapazitive Patch-Sensoreinheit weist insbesondere zumindest einen adhäsiven Klebestreifen bzw. zumindest einen Klebestreifen auf, der beispielsweise im Wesentlichen die Form eines Rechtecks oder eines Kreises aufweist. Eine kapazitive Patch-Sensoreinheit kann insbesondere in der Form einem medizinischen Pflaster ähneln. Beispielsweise weist ein im Wesentlichen rechteckiger adhäsiver Klebestreifen eine Länge und/oder Breite von jeweils etwa 0,5 cm bis etwa 10 cm, insbesondere von etwa 1,5 cm bis etwa 7 cm und bevorzugt von etwa 2 cm bis etwa 5 cm auf.

Die Messfläche und/oder die Klebefläche des Adhäsionsstreifens von kapazitiven Sensoreinheiten, bevorzugt von kapazitiven Patch-Sensoreinheiten, ist insbesondere geometrisch im Wesentlichen "unexzentrisch" gestaltet. Vorteilhafte Flächenformen können beispielsweise im Wesentlichen kreisförmig, quadratisch, rechteckig sein. Bei im Wesentlichen rechteckigen Messflächen ist ein bevorzugtes Kantenlängenverhältnis ein Wert von etwa unter 2, wie beispielsweise etwa 1 für im Wesentlichen quadratische Flächen oder etwa 1,5 oder etwa 0,5 für rechteckige Flächen, die keine quadratische Form aufweisen.

Eine kapazitive Bogen-Sensoreinheit weist insbesondere zumindest teilweise eine im Wesentlichen runde Form, insbesondere eine kreisrunde Bogenform auf. Beispielsweise kann die kapazitive Bogen-Sensoreinheit eine Form eines HalbkreisBogens aufweisen. Dabei hat die Form insbesondere einen gebogenen Streifen, der eine Breite von etwa 0,3 cm bis etwa 10 cm, insbesondere von etwa 1 cm bis etwa 7 cm und bevorzugt von etwa 1,5 cm bis etwa 3 cm aufweist, sodass die kapazitive Bogen-Sensoreinheit beispielsweise einen Port umschließen kann. Der Radius eines Kreisbogens einer kapazitiven Bogen-Sensoreinheit kann insbesondere zwischen etwa 0,5 cm und etwa 10 cm, insbesondere zwischen etwa 1 cm und etwa 7 cm und bevorzugt zwischen etwa 1,5 cm und etwa 3 cm liegen. Insbesondere weist die kapazitive Bogen-Sensoreinheit einen im Wesentlichen geraden Anschlussabschnitt auf.

Eine Sensoreinheit ist insbesondere eine kapazitive Einweg-Sensoreinheit zum im Wesentlichen einmaligen Anordnen an eine Bioreaktoranlage. Alternativ kann die kapazitive Sensoreinheit auch eine kapazitive Mehrweg-Sensoreinheit, die dazu ausgelegt ist, mehrfach an einer Bioreaktoranlage angeordnet zu werden.

Bei einer Sensoreinheit kann es sich um einen sogenannten "Füllstandschalter" handeln. Bei der Messung mit kapazitiven Schaltern wird eine Änderung der Dielektrizitätskonstante εᵣ erfasst und diese Änderung in ein Schaltsignal umgewandelt. Der Vorteil dieser Technologie liegt darin, dass das Medium beispielsweise hinter einer dielektrischen Behälterwand erfasst werden kann.

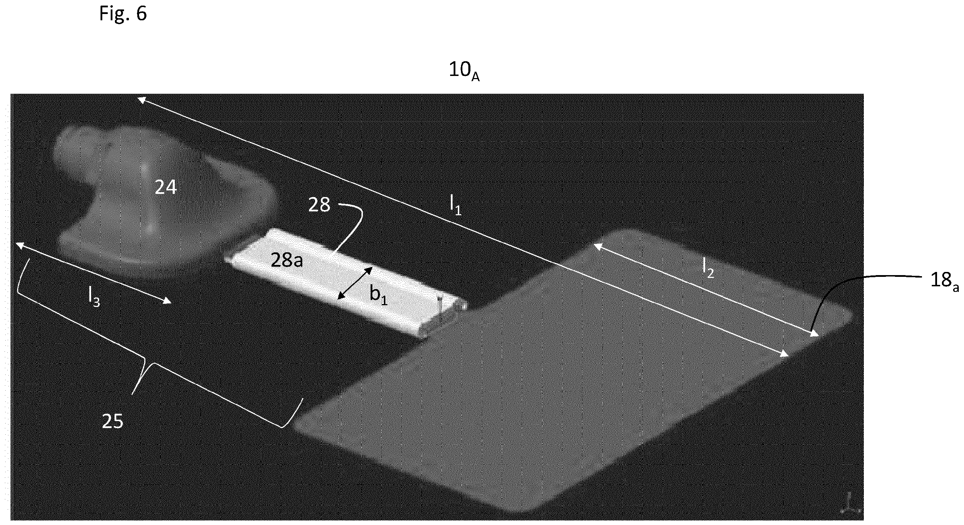

**Fig.** 6 ist eine schematische perspektivische Darstellung einer beispielhaften kapazitiven Patch-Sensoreinheit 10_{A} angebrachtem Abstandshalter.

Die kapazitive Patch-Sensoreinheit 10_{A} weist einen Anschlussabschnitt 25 mit einer Anschlusseinheit 24 und einer Konnektierungslippe 28 auf. Die Konnektierungslippe 28 stellt einen im Wesentlichen länglichen verbindenden Abschnitt dar, insbesondere eine elektrische Leiterbahn zur Versorgung des Elektrodensystems 19 mit Strom, an deren einen Ende sich das rechteckige Feld 18ₐ befindet und an deren anderen Ende sich die Anschlusseinheit 24 befindet. Die Anschlusseinheit 24 dient dabei beispielsweise der Signalübertragung und/oder der Spannungsversorgung. Beispielsweise kann derart durch eine angelegte Wechselspannung ein elektrisches Wechselfeld in dem Elektrodensystem 19 erzeugt und der resultierende Stromfluss als Signal detektiert werden.

Eine Konnektierungslippe 28 ist vorteilhaft, um den Anschluss bzw. den Anschlussabschnitt zwischen dem rechteckigen Feld 18ₐ der Patch-Sensoreinheit 10_{A} und dem Transmitterkabel (nicht gezeigt), das an der Anschlusseinheit 24 angeschlossen werden oder sein kann, flexibel zu halten. In anderen Worten wird eine im Wesentlichen flexible Konnektierungslippe 28 zwischen der Anschlusseinheit 24 und dem rechteckigen Feld 18ₐ der Patch-Sensoreinheit 10_{A} bereitgestellt. Ferner kann die Anschlusseinheit 24 bzw. der Konnektor, sofern die Anschlusseinheit 24 abnehmbar bzw. entfernbar ist, demzufolge auch einfach mit dem rechteckigen Feld 18ₐ der Patch-Sensoreinheit 10_{A} verbunden oder getrennt werden. Darüber hinaus ist die Konnektierungslippe 28 auch vorteilhaft, um die spezifizierte Grundkapazität der Patch-Sensoreinheit 10_{A} in einen gewünschten Bereich zu bringen, indem sie die Leiterbahnen der Patch-Sensoreinheit verlängert.

Insbesondere hat die Konnektierungslippe 28 keine definierte, das heißt dauerhaft konstante und vorbestimmte Position wie beispielsweise bevorzugt das rechteckige Feld 18ₐ der Patch-Sensoreinheit 10_{A}, da die Konnektierungslippe 28 bevorzugt nicht auf den Behälter aufgeklebt wird. Dadurch können unerwünschte Störsignale, beispielsweise falsch-positive Signale erzeugt und mittels des Elektrodensystems erfasst werden, insbesondere dann, wenn die Konnektierungslippe 28 im Prozessverlauf bewegt wird, wie z.B. durch Zug am Kabel oder Druckvariation im Bioreaktor.

Die Konnektierungslippe 28 sollte allerdings bevorzugt nicht an dem Behälter bewegungseinschränkend fixiert, insbesondere nicht geklebt werden, da die Konnektierungslippe 28 in dem Fall nicht die benötigte Flexibilität und Bewegungsfreiheit bereitstellt und sich die Konnektierungslippe 28 ohnehin durch die Bewegung des Kabels ggf. auch wieder leicht ablösen könnte. Somit können Störsignale insbesondere nicht dadurch verhindert oder reduziert werden, dass die Konnektierungslippe 28 an dem Behälter fixiert, insbesondere festgeklebt wird.

Bevorzugt wird der Abstandshalter 28a an der sensiblen Fläche, insbesondere an der Konnektierungslippe 28 bereitgestellt. Insbesondere stellt der Abstandshalter 28a einen Mantel aus Polypropylen dar. Alternativ können auch andere Kunststoffe verwendet werden. Dies hat den Vorteil, dass zuvor beschriebene Störsignale reduziert oder gar vermieden werden können.

Das Material des Abstandshalters kann bevorzugt zumindest teilweise eine Dicke von etwa 1 mm aufweisen, um einerseits im Wesentlichen flexibel zu sein und andererseits einen ausreichend großen Abstand von möglichen Fremdkörpern zum sensitiven Bereich der Konnektierungslippe zu gewährleisten.

Der Abstandshalter 28a deckt insbesondere einen Großteil der sensitiven Fläche ab. Der Abstandshalter 28a kann über einen Steg an einem Overmoulding des Konnektors ausgerichtet werden. Der Verschluss ist bevorzugt reversibel verschließbar, damit der Abstandshalter mehrfach genutzt werden kann. Der Abstandshalter hat abgerundete bzw. entgratete Kanten, um insbesondere nicht in einen Einweg-Behälter einzuschneiden und eine Dünnstelle damit sie einteilig ausgestaltet sein kann und die Konnektierungslippe 28 wie eine Lasche umschließen kann.

Die Offenbarung betrifft somit insbesondere eine kapazitive Patch-Sensoreinheit 10_{A} mit:
- einem Feld, insbesondere ein rechteckiges Feld 18ₐ zum Anbringen an einer Oberfläche;
- einem Elektrodensystem 19, das zumindest teilweise an dem Feld angeordnet ist und dazu ausgelegt ist, Änderungen der Permittivität und/oder der Kapazität in der unmittelbaren Nähe bzw. Umgebung zu erfassen und insbesondere zum kapazitiven Messen an einer der Anordnungspositionen der Bioreaktoranlage 50 und zum Erfassen des Vorhandenseins von Schaum 9₂, 9₃ an der Anordnungsposition A1-A3; B1-B6 auf Basis der kapazitiven Messung;;
- einer Konnektierungslippe 28, zum elektrischen Verbinden eines Netzanschlusses mit dem Elektrodensystem; und bevorzugt
- einen Abstandshalter 28a, der dazu ausgelegt ist, die Konnektierungslippe 28 zumindest teilweise zu umschließen und/oder abzudecken.

Die Offenbarung betrifft auch insbesondere eine kapazitive Bogen-Sensoreinheit 10_{B} mit:
- einem Feld, insbesondere einen Bogenabschnitt 18_{b} zum Anbringen an einer Oberfläche;
- einem Elektrodensystem 19, das zumindest teilweise an dem Feld angeordnet ist und dazu ausgelegt ist, Änderungen der Permittivität und/oder der Kapazität in der unmittelbaren Nähe bzw. Umgebung zu erfassen und insbesondere zum kapazitiven Messen an einer der Anordnungspositionen der Bioreaktoranlage 50 und zum Erfassen des Vorhandenseins von Schaum 9₂, 9₃ an der Anordnungsposition A1-A3; B1-B6 auf Basis der kapazitiven Messung;
- einer Konnektierungslippe 28, zum elektrischen Verbinden eines Netzanschlusses mit dem Elektrodensystem; und bevorzugt
- einen Abstandshalter 28a, der dazu ausgelegt ist, die Konnektierungslippe 28 zumindest teilweise zu umschließen und/oder abzudecken.

Im Allgemeinen kann eine Sensoreinheit ein Feld jeder denkbaren Form aufweisen, wobei das Feld an einer Oberfläche der Bioreaktoranlage angebracht werden kann. Eine solche Sensoreinheit kann eine Konnektierungslippe und einen Abstandshalter aufweisen, ganz unabhängig von der Form des besagten Feldes. Die Konnektierungslippe ist insbesondere so ausgelegt, dass sie nicht an der Oberfläche der Bioreaktoranlage fixiert und/oder angeklebt wird. Die Konnektierungslippe verbindet ein Elektrodensystem, das an dem Feld angeordnet ist mit einer Anschlusseinheit, bevorzugt elektrisch.

| | |
|---|---|
| 1 | Einwegbehälter eines Bioreaktors |
| 1a | Behälterhüllen-Innenseite |
| 1b | Behälterhüllen-Außenseite |
| 2 | Behälter-Innenraum bzw. Innenraum des Einwegbehälters |
| 3 | Behälter-Innendecke |
| 4 | Behälter-Innenboden |
| 5 | Behälter-Längsachse |
| 6 | Grenzlinie für maximalen Füllstand |
| 7 | Grenzlinie für minimalen Füllstand |
| 8₁, 8₂ | Kabelport |
| 9 | Medium bzw. Bioreaktor-Medienfüllung |
| 9₁ | Fluides Medium |
| 9₂ | Schaumiges Medium/Schaum auf fluidem Medium |
| 9₃ | Schaumiges Medium/Schaum als Ablagerung an der Behälterhüllen-Innenseite |
| 10_{A} | kapazitive (Patch-) Sensoreinheit |
| 10_{A1}, 10_{A2}, 10_{A3} | Erste, zweite und dritte kapazitive Patch-Sensoreinheit zur Erfassung des Niveaus eines Schaums, insbesondere von der Behälterhüllen-Innenseite |
| 10_{A'6,} 10_{A'1} | kapazitive Patch-Sensoreinheit zur Erfassung des Niveaus eines Schaums, insbesondere von der Behälterhüllen-Außenseite |
| 10_{B} | kapazitive (Bogen-) Sensoreinheit |
| 10_{B2,} 10_{B3} | Erste und zweite kapazitive Bogen-Sensoreinheit zur Erfassung des Vorhandenseins von Schaum mit Außenanbringung |
| 10_{C1,} 10_{C2} | Manschetten-Sensoreinheiten, welche zumindest teilweise um ein Rohr und/oder einen Schlauch gelegt werden können |
| 11 | Kabel |
| 12a | Abluftschlauch |
| 12b | Abluftport bzw. Port für Abluftschlauch |
| 12c | Filter |
| 13a | Rohr oder Schlauch für Ober-Zugang/Ablauf |
| 13b | Port für Ober-Zugang/Ablauf |
| 14 | Kontrolleinheit |
| 15 | Recheneinheit |
| 16 | Steuer- und/oder Regeleinheit |
| 17 | Rohr oder Schlauch zum Steuern und/oder Regeln der Schaumbildung bzw. des Schaumniveaus |
| 18 | Adhäsiver Klebestreifen |
| 18a | Rechteckiges Feld |
| 18_{b} | Bogenabschnitt |
| 19 | Elektrodensystem |
| 20 | Sensorelektrode |
| 21 | Schutzelektrode |
| 22 | Masseelektrode |
| 23 | Substanz zum Vermindern und/oder Verhindern von Schaum(bildung) bzw. Antischaum-Mittel |
| 24 | Anschlusseinheit |
| 25 | Anschlussabschnitt |
| 26 | Rühreinheit |
| 28 | Konnektierungslippe des Sensorabschnitts 25 |
| 28a | Abstandshalter |
| 50 | Bioreaktoranlage |
| 100 | System zum Erfassen zumindest eines Vorhandenseins von Schaum |
| A1, A2, A3 | Erste, zweite und dritte Anordnungsposition für eine kapazitive Sensoreinheit zur Erfassung des Niveaus eines Schaums, insbesondere von der Behälterhüllen-Innenseite |
| AR | Außenraum des Behälters |
| B1, B2, B3, C1, C2, B6 | Erste bis sechste Anordnungsposition für eine kapazitive Sensoreinheit zur Erfassung des Vorhandenseins, insbesondere des Niveaus eines Schaums, insbesondere von der Behälterhüllen-Außenseite |
| bₐ | Breite des rechteckigen Feldes |
| b_{b} | Breite des rechteckigen Feldes |
| b_{c} | Breite des Streifens des Bogenabschnitts |
| b_{d} | Mindestbreite des Bogens, der Elektroden |
| bₑ | Gesamtbreite des Bogenabschnitts |
| h₅ | Höhe vom Bioreaktor-Innenboden zur Unterkante der zweiten kapazitiven Patch-Sensoreinheit 10_{A2} |
| h₆ | Höhe vom Bioreaktor-Innenboden zum tatsächlichen Füllstand der fluiden Bioreaktor-Medienfüllung bzw. Höhe der Flüssigkeit |
| h₇ | Höhe vom Bioreaktor-Innenboden zur Unterkante der dritten kapazitiven Patch-Sensoreinheit 10_{A3} |
| h₈ | Höhe vom Bioreaktor-Innenboden zum tatsächlichen Füllstand der schaumigen Bioreaktor-Medienfüllung bzw. Höhe des Schaums |
| IR | Innenraum des Schlauchs |
| I₁ | Gesamtlänge inklusive Anschlussabschnitt |
| I₂ | Länge des rechteckigen Feldes |
| I₃ | Länge der Anschlusseinheit |
| I₄ | Länge das Anschlussabschnitts |
| I₅ | Gesamtlänge inklusive Anschlussabschnitt |
| R | Radius |

## Patentansprüche

1. System (100) zum Erfassen zumindest eines Vorhandenseins von Schaum (9₂, 9₃) eines Mediums (9) in einer Bioreaktoranlage (50), wobei das System (100) umfasst:
- eine Bioreaktoranlage (50) mit zumindest einem Einwegbehälter (1) zum Aufnehmen des Mediums (9), das den Schaum (9₂, 9₃) aufweisen kann, und einem Abluftport (12b);
- zumindest zwei kapazitive Sensoreinheiten (10_{A}, 10_{B} ,10_{C}), die an zumindest zwei unterschiedlichen Anordnungspositionen (A1-A3; B1-B6; C1, C2) der Bioreaktoranlage (50) angebracht sind, wobei eine Sensoreinheit (10_{A}, 10_{B} ,10c) der zumindest zwei kapazitiven Sensoreinheiten (10_{A}, 10_{B} ,10_{C}):
an einer ersten Anordnungsposition (B2) in unmittelbarer Nähe des Abluftports (12b) oder
an einer zweiten Anordnungsposition (C1) an oder in räumlicher Nähe zu einem Abluftschlauch (12a) der Bioreaktoranlage (50), wobei der Abluftschlauch (12a) fluidisch mit dem Abluftport (12b) verbunden ist, angeordnet ist; und
- zumindest eine Kontrolleinheit (14) mit zumindest einer Steuer- und/oder Regeleinheit (16), wobei die Kontrolleinheit (14) eingerichtet ist bei Auslösen der an der ersten Anordnungsposition (B2) oder der zweiten Anordnungsposition (C1) angeordneten Sensoreinheit (10_{A}, 10_{B} ,10c) eine Maßnahme wie eine Abschaltung eines Rührers und/oder einer Begasung auszulösen,
wobei die kapazitiven Sensor-Einheiten (10_{A}, 10_{B} ,10c) jeweils zumindest ein Elektrodensystem (19) für eine kapazitive Messung aufweisen und das Vorhandensein von Schaum (9₂, 9₃) an den zumindest zwei Anordnungspositionen (A1-A3; B1-B6) auf Basis der kapazitiven Messung erfassen können; und
wobei die kapazitive Sensor-Einheiten (10_{A}, 10_{B} ,10c) dazu ausgelegt sind, erfasste Daten betreffend das Vorhandensein von Schaum (9₂, 9₃) an die zumindest eine Kontrolleinheit (14) zum Kontrollieren, Steuern und/oder Regeln einer Schaumbildung (9) in der Bioreaktoranlage (50) auf deren Basis zu übermitteln.

2. System (100) nach Anspruch 1, wobei zumindest eine der kapazitiven Sensoreinheiten (10_{A}, 10_{B}) einen adhäsiven Klebestreifen (18) aufweist, an dem das Elektrodensystem (19) zumindest teilweise angeordnet ist, wobei der adhäsive Klebestreifen (18) dazu ausgelegt ist, dass die zumindest eine kapazitive Sensoreinheit (10_{A}, 10_{B}) an der Bioreaktoranlage (50) angeordnet werden kann.

3. System (100) nach Anspruch 1 oder 2, wobei zumindest eine der kapazitiven Sensoreinheiten (10_{A}, 10_{B} ,10_{C}) eine kapazitive Bogen-Sensoreinheit (10_{B}) darstellt, wobei die Form der kapazitiven Bogen-Sensoreinheit (10_{B}) zumindest einen Abschnitt eines Bogens, insbesondere eines Kreisbogens umfasst.

4. System (100) nach Anspruch 3, wobei die Bioreaktoranlage (50) zumindest einen Port (12b, 13b), insbesondere einen kreisförmigen Port (12b, 13b) zum fluidischen Verbinden des Innenraums (2) des Einwegbehälters (1) mit einem anderen Element und/oder dem Außenraum (AR) des Einwegbehälters (1) aufweist und wobei die zumindest eine kapazitive Bogen-Sensoreinheit (10_{B}) dazu ausgelegt ist, den Port (12b, 13b) zumindest teilweise zu umschließen.

5. System (100) nach Anspruch 4, wobei die Bioreaktoranlage (50) zumindest einen fluidisch mit dem Port (12b, 13b) verbundenen Schlauch (12a, 13a) und/oder ein Rohr aufweist und wobei die zumindest eine kapazitive Bogen-Sensoreinheit (10_{B}) dazu ausgelegt ist, den Schlauch (12a, 13a) und/oder das Rohr zumindest teilweise zu umschließen.

6. System (100) nach einem der vorangehenden Ansprüche, wobei zumindest eine der kapazitiven Sensoreinheiten (10_{A1-A3}), insbesondere eine kapazitive Patch-Sensoreinheit (10_{A1-A3}) dazu ausgelegt ist, das Vorhandensein von Schaum, insbesondere ein Schaumniveau in dem Einwegbehälter zu erfassen und an einer Anordnungsposition (A1, A2, A3) im Innenraum (2) des Einwegbehälters (1), insbesondere an einer Behälterhüllen-Innenseite (1a) angeordnet ist und wobei bevorzugt die zumindest eine kapazitive Sensoreinheit (10_{A}) sterilisierbar, insbesondere sterilisiert ist.

7. System (100) nach einem der vorangehenden Ansprüche, wobei zumindest eine der kapazitiven Sensoreinheiten, insbesondere eine kapazitive Bogen-Sensoreinheit (10_{B2,B3}) und/oder eine Manschetten-Sensoreinheit (10_{C1}, 10_{C2}) an einer Anordnungsposition (B2, B3, C1, C2) außerhalb des Einwegbehälters (1), insbesondere an einer Behälterhüllen-Außenseite (1b) und/oder an einem Port (12b, 13b) und/oder an einem Schlauch bzw. Rohr bzw. Leitung (12a, 13a) angeordnet werden kann oder angeordnet ist.

8. System (100) nach Anspruch 7, wobei die Anordnungsposition (B1-B3, B6, C1, C2) an der Behälterhüllen-Außenseite (1a) zumindest eine der folgenden Anordnungspositionen darstellt:
- Anordnungsposition (B1) auf einer Höhe (h₉) knapp unterhalb der Höhe (hs) des Abluftports (12b) des Einwegbehälters (1), insbesondere nicht in unmittelbarer Nähe des Abluftports (12b);
- Anordnungsposition (B3) in unmittelbarer Nähe eines Ports (13b) für einen Ober-Zugang/Ablauf (13a) des Einwegbehälters (1);
- Anordnungsposition (C2) an dem Abluftschlauch (12a) der Bioreaktoranlage (50) in unmittelbarer Nähe eines Filters (12c) des Abluftschlauchs (12a), insbesondere zwischen dem Abluftport (12b) und dem Filter (12c).

9. System (100) nach einem der Ansprüche 6 bis 8, wobei die Anordnungsposition (A1, A2, A3) an der Behälterhüllen-Innenseite (1a) zumindest eine der folgenden Anordnungspositionen darstellt:
- Anordnungsposition (A1) in der Nähe, bevorzugt auf einer Höhe (hs) knapp oberhalb einer Höhe (h₂) eines vorbestimmten maximalen Füllstandes des Einwegbehälters (1), insbesondere oberhalb einer Grenzlinie (6) des vorbestimmten maximalen Füllstandes;
- Anordnungsposition (A2) in der Nähe, bevorzugt auf einer Höhe (hs) knapp oberhalb einer Höhe (h₄) eines vorbestimmten minimalen Füllstandes des Einwegbehälters (1), insbesondere einer Grenzlinie (7) des vorbestimmten minimalen Füllstandes;
- Anordnungsposition (A3) auf einer Höhe (h₇) zwischen der Höhe (h₄) des vorbestimmten minimalen Füllstandes und der Höhe (h₂) des vorbestimmten maximalen Füllstandes, insbesondere zwischen einer Grenzlinie (7) des vorbestimmten minimalen Füllstandes und einer Grenzlinie (6) des vorbestimmten maximalen Füllstandes.

10. Verfahren zum Erfassen zumindest eines Vorhandenseins von Schaum (9₂, 9₃) eines Mediums (9), umfassend:
- Bereitstellen einer Bioreaktoranlage (50) mit zumindest einem Einwegbehälter (1) zum Aufnehmen des Mediums (9), das den Schaum (9₂, 9₃) umfassen kann, und einem Abluftport (12b);
- Anordnen an mindestens zwei Anordnungspositionen (A1-A3; B1-B6; C1, C2) der Bioreaktoranlage (50) zumindest zweier kapazitiver Sensoreinheiten (10_{A}, 10_{B} ,10_{C}), wobei die kapazitiven Sensoreinheiten jeweils zumindest ein Elektrodensystem (19) für eine kapazitive Messung aufweisen und wobei eine Sensoreinheit (10_{A}, 10_{B} ,10c) der zumindest zwei kapazitiven Sensoreinheiten (10_{A}, 10_{B} ,10_{C}):
an einer ersten Anordnungsposition (B2) in unmittelbarer Nähe des Abluftports (12b) oder
an einer zweiten Anordnungsposition (C1) an oder in räumlicher Nähe zu einem Abluftschlauch (12a) der Bioreaktoranlage (50), wobei der Abluftschlauch (12a) fluidisch mit dem Abluftport (12b) verbunden ist, angeordnet ist;
- Erfassen von Daten betreffend das Vorhandensein von Schaum an den zumindest zwei Messpositionen auf Basis der kapazitiven Messungen; und
- Übermitteln der erfassten Daten betreffend das Vorhandensein von Schaum (9₂, 9₃) an zumindest eine Kontrolleinheit (14) zum Kontrollieren, Steuern und/oder Regeln einer Schaumbildung (9) in der Bioreaktoranlage (50) auf deren Basis mittels der kapazitiven Sensor-Einheiten (10_{A}, 10_{B} ,10c),
- Auslösen einer Maßnahme wie eine Abschaltung eines Rührers und/oder einer Begasung bei Auslösen der zweiten Sensoreinheit (10_{A}, 10_{B} ,10_{C}).

11. Verfahren nach Anspruch 10, umfassend Überwachen, Steuern und/oder Regeln der Schaumbildung (9) in der Bioreaktoranlage (50) mittels der Kontrolleinheit (14) auf Basis der übermittelten Daten.

12. Verfahren nach Anspruch 10 oder 11, wobei das Steuern und/oder Regeln ein Zuführen einer Substanz (23) umfasst, die dazu ausgelegt ist, eine Schaumbildung zu verhindern oder zumindest zu vermindern, wenn das Vorhandensein von Schaum mittels zumindest einer kapazitiven Sensoreinheit erfasst wird, bevorzugt, wenn das Vorhandensein von Schaum mittels zumindest einer kapazitiven Sensoreinheit an einer Anordnungsposition außerhalb des Einwegbehälters, insbesondere mittels einer kapazitiven Bogen-Sensoreinheit erfasst wird.

13. Verfahren nach einem der Ansprüche 10 bis 12, umfassend:
- Auslösen eines Alarms wenn das Vorhandensein von Schaum mittels zumindest einer kapazitiven Sensoreinheit (10_{A}, 10_{B} ,10c) an einer Anordnungsposition (A1-A3) im Innenraum (2) des Einwegbehälters (1) und/oder an einer Anordnungsposition (B1-B6) außerhalb des Einwegbehälters (1) erfasst wird; und/oder
- Auslösen eines Notfall-Aus wenn das Vorhandensein von Schaum mittels zumindest eines Sensors (10_{A}, 10_{B} ,10c) an einer Anordnungsposition (B1-B6) außerhalb des Einwegbehälters (1) erfasst wird.

14. Verfahren zur Montage eines Systems (100) nach einem der Ansprüche 1 bis 9 zum Erfassen zumindest eines Vorhandenseins von Schaum (9₂, 9₃) eines Mediums (9) in einer Bioreaktoranlage (50) umfassend:
- Bereitstellen einer Bioreaktoranlage (50) mit zumindest einem Einwegbehälter (1) zum Aufnehmen des Mediums (9), das den Schaum (9₂, 9₃) umfassen kann;
- Bereitstellen zumindest zweier kapazitiver Sensoreinheiten (10_{A}, 10_{B} ,10c), wobei die kapazitiven Sensoreinheiten jeweils zumindest ein Elektrodensystem (19) für eine kapazitive Messung aufweisen;
- Anordnen der zumindest zwei kapazitiven Sensoreinheiten (10_{A}, 10_{B} ,10c) an jeweils einer Anordnungsposition (A1-A3; B1-B6; C1, C2) der Bioreaktoranlage (50); und
- Anschließen der zumindest zwei kapazitiven Sensoreinheiten (10_{A}, 10_{B} ,10c) an zumindest eine Kontrolleinheit (14) zum Kontrollieren, Steuern und/oder Regeln einer Schaumbildung (9) in der Bioreaktoranlage (50) auf Basis der kapazitiven Messungen.

## Claims

1. System (100) for detecting at least one presence of foam (9₂, 9₃) of a medium (9) in a bioreactor plant (50), wherein the system (100) comprises:
- a bioreactor plant (50) comprising at least one single-use container (1) for receiving the medium (9) that may comprise the foam (9₂, 9₂), and an exhaust port (12b) ;
- at least two capacitive sensor units (10_{A}, 10_{B}, 10_{C}) which are attached at at least two different situating positions (A1-A3; B1-B6; C1, C2) of the bioreactor plant (50), wherein one sensor unit (10_{A}, 10_{B}, 10_{C}) of the at least two capacitive sensor units (10_{A}, 10_{B}, 10_{C}) is arranged:
at a first situating position (B2) in the immediate vicinity of the exhaust port (12b) or
at a second situating position (C1) on or in the spatial vicinity of an exhaust hose (12a) of the bioreactor plant (50), wherein the exhaust hose (12a) is fluidically connected to the exhaust port (12b); and
- at least one monitoring unit (14) comprising at least one open-loop and/or closed-loop control unit (16), wherein the monitoring unit (14) is designed to trigger an action, such as switching off a stirrer and/or a sparging, when the sensor unit (10_{A}, 10_{B}, 10_{C}) arranged at the first situating position (B2) or the second situating position (C1) is triggered,
wherein the capacitive sensor units (10_{A}, 10_{B}, 10_{C}) each comprise at least one electrode system (19) for a capacitive measurement and can detect the presence of foam (9₂, 9₃) at the at least two situating positions (A1-A3; B1-B6) on the basis of the capacitive measurement; and
wherein the capacitive sensor units (10_{A}, 10_{B}, 10_{C}) are designed to transmit recorded data concerning the presence of foam (9₂, 9₃) to the at least one monitoring unit (14) for monitoring, open-loop control and/or closed-loop control of foam formation (9) in the bioreactor plant (50) on the basis of said data.

2. System (100) according to claim 1, wherein at least one of the capacitive sensor units (10_{A}, 10_{B}) comprises an adhesive strip (18), on which the electrode system (19) is at least partially arranged, wherein the adhesive strip (18) is designed such that the at least one capacitive sensor unit (10_{A}, 10_{B}) can be arranged on the bioreactor plant (50).

3. System (100) according to claim 1 or 2, wherein at least one of the capacitive sensor units (10_{A}, 10_{B}, 10_{C}) is a capacitive arc sensor unit (10_{B}), wherein the shape of the capacitive arc sensor unit (10_{B}) comprises at least a section of an arc, in particular of a circular arc.

4. System (100) according to claim 3, wherein the bioreactor plant (50) has at least one port (12b, 13b), in particular a circular port (12b, 13b), for fluidically connecting the interior (2) of the single-use container (1) to another element and/or to the exterior (AR) of the single-use container (1), and wherein the at least one capacitive arc sensor unit (10_{B}) is designed to at least partially encircle the port (12b, 13b).

5. System (100) according to claim 4, wherein the bioreactor plant (50) has at least one hose (12a, 13a) and/or tube fluidically connected to the port (12b, 13b), and wherein the at least one capacitive arc sensor unit (10_{B}) is designed to at least partially encircle the hose (12a, 13a) and/or tube.

6. System (100) according to any of the preceding claims, wherein at least one of the capacitive sensor units (10_{A1-A3}), in particular a capacitive patch sensor unit (10_{A1-A3}), is designed to detect the presence of foam, in particular a foam level, in the single-use container and is arranged at a situating position (A1, A2, A3) in the interior (2) of the single-use container (1), in particular on an interior container envelope (1a), and wherein preferably the at least one capacitive sensor unit (10_{A}) can be sterilized, in particular is sterilized.

7. System (100) according to any of the preceding claims, wherein at least one of the capacitive sensor units, in particular a capacitive arc sensor unit (10_{B2,B3}) and/or a collar sensor unit (10_{C1}, 10_{C2}) , can be arranged or is arranged at a situating position (B2, B3, C1, C2) outside of the single-use container (1), in particular on an exterior container envelope (1b) and/or on a port (12b, 13b) and/or on a hose or tube or line (12a, 13a).

8. System (100) according to claim 7, wherein the situating position (B1-B3, B6, C1, C2) on the exterior container envelope (1a) is at least one of the following situating positions:
- situating position (B1) at a height (h₉) slightly below the height (h₈) of the exhaust port (12b) of the single-use container (1), in particular not in the immediate vicinity of the exhaust port (12b);
- situating position (B3) in the immediate vicinity of a port (13b) for an upper inlet/outlet (13a) of the single-use container (1);
- situating position (C2) on the exhaust hose (12a) of the bioreactor plant (50) in the immediate vicinity of a filter (12c) of the exhaust hose (12a), in particular between the exhaust port (12b) and the filter (12c).

9. System (100) according to any of claims 6 to 8, wherein the situating position (A1, A2, A3) on the interior container envelope (1a) is at least one of the following situating positions:
- situating position (A1) in the vicinity of, preferably at a height (h₃) slightly above a height (h₂) of, a predetermined maximum fill level of the single-use container (1), in particular above a limit line (6) of the predetermined maximum fill level;
- situating position (A2) in the vicinity of, preferably at a height (h₅) slightly above a height (h₄) of, a predetermined minimum fill level of the single-use container (1), in particular a limit line (7) of the predetermined minimum fill level;
- situating position (A3) at a height (h₇) between the height (h₄) of the predetermined minimum fill level and the height (h₂) of the predetermined maximum fill level, in particular between a limit line (7) of the predetermined minimum fill level and a limit line (6) of the predetermined maximum fill level.

10. Method for detecting at least one presence of foam (9₂, 9₃) of a medium (9), which method comprises:
- providing a bioreactor plant (50) comprising at least one single-use container (1) for receiving the medium (9) that may comprise the foam (9₂, 9₃), and an exhaust port (12b);
- arranging at least two capacitive sensor units (10_{A}, 10_{B}, 10_{C}) at at least two situating positions (A1-A3; B1-B6; C1, C2) of the bioreactor plant (50), wherein the capacitive sensor units each comprise at least one electrode system (19) for a capacitive measurement, and wherein one sensor unit (10_{A}, 10_{B}, 10_{C}) of the at least two capacitive sensor units (10_{A}, 10_{B}, 10_{C}) is arranged:
at a first situating position (B2) in the immediate vicinity of the exhaust port (12b) or
at a second situating position (C1) on or in the spatial vicinity of an exhaust hose (12a) of the bioreactor plant (50), wherein the exhaust hose (12a) is fluidically connected to the exhaust port (12b);
- recording data concerning the presence of foam at the at least two measurement positions on the basis of the capacitive measurements; and
- transmitting the recorded data concerning the presence of foam (9₂, 9₃) to at least one monitoring unit (14) for monitoring, open-loop control and/or closed-loop control of foam formation (9) in the bioreactor plant (50) on the basis of said data, by means of the capacitive sensor units (10_{A}, 10_{B}, 10_{C}),
- triggering an action, such as switching off a stirrer and/or a sparging, when the second sensor unit (10_{A}, 10_{B}, 10_{C}) is triggered.

11. Method according to claim 10, which comprises monitoring, open-loop control and/or closed-loop control of foam formation (9) in the bioreactor plant (50) by means of the monitoring unit (14) on the basis of the transmitted data.

12. Method according to claim 10 or 11, wherein the open-loop control and/or closed-loop control comprises feeding in a substance (23), which is designed to prevent or at least reduce foam formation, when the presence of foam is detected by at least one capacitive sensor unit, preferably when the presence of foam is detected by at least one capacitive sensor unit at a situating position outside of the single-use container, in particular by a capacitive arc sensor unit.

13. Method according to any of claims 10 to 12, which comprises:
- triggering an alarm when the presence of foam is detected by at least one capacitive sensor unit (10_{A}, 10_{B}, 10_{C}) at a situating position (A1-A3) in the interior (2) of the single-use container (1) and/or at a situating position (B1-B6) outside of the single-use container (1); and/or
- triggering an emergency shut-off when the presence of foam is detected by at least one sensor (10_{A}, 10_{B}, 10_{C}) at a situating position (B1-B6) outside of the single-use container (1).

14. Method for installing a system (100) according to any of claims 1 to 9 for detecting at least one presence of foam (9₂, 9₃) of a medium (9) in a bioreactor plant (50), which method comprises:
- providing a bioreactor plant (50) comprising at least one single-use container (1) for receiving the medium (9) that may comprise the foam (9₂, 9₃);
- providing at least two capacitive sensor units (10_{A}, 10_{B}, 10_{C}), wherein the capacitive sensor units each comprise at least one electrode system (19) for a capacitive measurement;
- arranging the at least two capacitive sensor units (10_{A}, 10_{B}, 10_{C}) at respective situating positions (A1-A3; B1-B6; C1, C2) of the bioreactor plant (50); and
- connecting the at least two capacitive sensor units (10_{A}, 10_{B}, 10_{C}) to at least one monitoring unit (14) for monitoring, open-loop control and/or closed-loop control of foam formation (9) in the bioreactor plant (50) on the basis of the capacitive measurements.

## Revendications

1. Système (100) pour la détection d'au moins une présence de mousse (9₂, 9₃) d'un milieu (9) dans une installation de bioréacteur (50), le système (100) comprenant :
- une installation de bioréacteur (50) pourvue d'au moins un récipient à usage unique (1) pour la réception du milieu (9), qui peut présenter la mousse (9₂, 9₃), et d'un orifice d'air d'échappement (12b) ;
- au moins deux unités de capteur capacitif (10_{A}, 10_{B}, 10_{C}), qui sont agencées à au moins deux emplacements de disposition différents (A1-A3 ; B1-B6 ; C1, C2) de l'installation de bioréacteur (50), une unité de capteur (10_{A}, 10_{B},10_{C}) des au moins deux unités de capteur capacitif (10_{A}, 10_{B},10_{C}) étant disposée :
en un premier emplacement de disposition (B2) dans le voisinage immédiat de l'orifice d'air d'échappement (12b) ou
en un deuxième emplacement de disposition (C1) au ou dans le voisinage spatial d'un tuyau d'air d'échappement (12a) de l'installation de bioréacteur (50), le tuyau d'air d'échappement (12a) étant en liaison fluidique avec l'orifice d'air d'échappement (12b) ; et
- au moins une unité de contrôle (14) pourvue d'au moins une unité de commande et/ou de régulation (16), l'unité de contrôle (14) étant conçue pour déclencher une mesure telle qu'un arrêt d'un agitateur et/ou d'un gazage lors du déclenchement de l'unité de capteur (10_{A}, 10_{B}, 10_{C}) disposée en le premier emplacement de disposition (B2) ou le deuxième emplacement de disposition (C1),
dans lequel les unités de capteur capacitif (10_{A}, 10_{B},10_{C}) présentent respectivement au moins un système d'électrodes (19) pour une mesure capacitive et peuvent détecter la présence de mousse (9₂, 9₃) en les au moins deux emplacements de disposition (A1-A3 ; B1-B6) sur la base de la mesure capacitive ; et
dans lequel les unités de capteur capacitif (10_{A}, 10_{B},10_{C}) sont conçues pour transmettre les données captées concernant la présence de mousse (9₂, 9₃) à la au moins une unité de contrôle (14) pour le contrôle, la commande et/ou la régulation d'une formation de mousse (9) dans l'installation de bioréacteur (50) sur leur base.

2. Système (100) selon la revendication 1, dans lequel au moins l'une des unités de capteur capacitif (10_{A}, 10_{B}) présente un ruban adhésif (18) sur lequel est disposé au moins partiellement le système d'électrodes (19), le ruban adhésif (18) étant conçu pour que l'unité de capteur capacitif (10_{A}, 10_{B}) puisse être disposée sur l'installation de bioréacteur (50) .

3. Système (100) selon la revendication 1 ou 2, dans lequel au moins l'une des unités de capteur capacitif (10_{A}, 10_{B}, 10_{C}) constitue une unité de capteur en arc capacitif (10_{B}), la forme de l'unité de capteur en arc capacitif (10_{B}) comprenant au moins une section d'un arc, en particulier d'un arc de cercle.

4. Système (100) selon la revendication 3, dans lequel l'installation de bioréacteur (50) présente au moins un orifice (12b, 13b), en particulier un orifice circulaire (12b, 13b) pour la liaison fluidique de l'espace intérieur (2) du récipient à usage unique (1) avec un autre élément et/ou l'espace extérieur (AR) du récipient à usage unique (1) et la au moins une unité de capteur en arc capacitif (10_{B}) étant conçue pour entourer au moins partiellement l'orifice (12b, 13b).

5. Système (100) selon la revendication 4, dans lequel l'installation de bioréacteur (50) présente au moins un tuyau (12a, 13a) en liaison fluidique avec l'orifice (12b, 13b) et/ou un tube et dans lequel la au moins une unité de capteur en arc capacitif (10_{B}) est conçue de manière à entourer au moins partiellement le tuyau (12a, 13a) et/ou le tube.

6. Système (100) selon l'une des revendications précédentes, dans lequel au moins l'une des unités de capteur capacitif (10_{A1-A3}), en particulier une unité de capteur en patch capacitif (10_{A1-A3}), est conçue de manière à détecter la présence de mousse, en particulier d'un niveau de mousse dans le récipient à usage unique et est disposée à un emplacement de disposition (A1, A2, A3) dans l'espace intérieur (2) du récipient à usage unique (1), en particulier sur une face intérieure de l'enveloppe de récipient (1a), et dans lequel de préférence la au moins une unité de capteur capacitif (10_{A}) peut être stérilisée, est en particulier stérilisée.

7. Système (100) selon l'une des revendications précédentes, dans lequel au moins l'une des unités de capteur capacitif, en particulier une unité de capteur en arc capacitif (10_{B2,B3}) et/ou une unité de capteur manchette (10_{C1}, 10_{C2}) peut être disposée ou est disposée en un emplacement de disposition (B2, B3, C1, C2) en dehors du récipient à usage unique (1), en particulier sur une face extérieure de l'enveloppe de récipient (1b) et/ou au niveau d'un orifice (12b, 13b) et/ou d'un tuyau et/ou d'un tube et/ou d'une conduite (12a, 13a).

8. Système (100) selon la revendication 7, dans lequel l'emplacement de disposition (B1-B3, B6, C1, C2) sur la face extérieure de l'enveloppe de récipient (1a) constitue au moins l'un des emplacements de disposition suivants :
- un emplacement de disposition (B1) à une hauteur (h₉) juste un peu en dessous de la hauteur (h₈) de l'orifice d'air d'échappement (12b) du récipient à usage unique (1), en particulier pas dans le voisinage immédiat de l'orifice d'air d'échappement (12b) ;
- un emplacement de disposition (B3) dans le voisinage immédiat d'un orifice (13b) pour un accès/écoulement supérieur (13a) du récipient à usage unique (1) ;
- un emplacement de disposition (C2) au niveau du tuyau d'air d'échappement (12a) de l'installation de bioréacteur (50) dans le voisinage immédiat d'un filtre (12c) du tuyau d'air d'échappement (12a), en particulier entre l'orifice d'air d'échappement (12b) et le filtre (12c).

9. Système (100) selon l'une des revendications 6 à 8, dans lequel l'emplacement de disposition (A1, A2, A3) sur la face intérieure de l'enveloppe de récipient (1a) constitue au moins l'un des emplacements de disposition suivants :
- un emplacement de disposition (A1) à proximité, de préférence à une hauteur (h₃) juste un peu au-dessus d'une hauteur (h₂) d'un niveau de remplissage maximal prédéfini du récipient à usage unique (1), en particulier au-dessus d'une ligne limite (6) du niveau de remplissage maximal prédéfini ;
- un emplacement de disposition (A2) à proximité, de préférence à une hauteur (h₅) juste un peu au-dessus d'une hauteur (h₄) d'un niveau de remplissage minimal prédéfini du récipient à usage unique (1), en particulier d'une ligne limite (7) du niveau de remplissage minimal prédéfini ;
- un emplacement de disposition (A3) à une hauteur (h₇) entre la hauteur (h₄) du niveau de remplissage minimal prédéfini et la hauteur (h₂) du niveau de remplissage maximal prédéfini, en particulier entre une ligne limite (7) du niveau de remplissage minimal prédéfini et une ligne limite (6) du niveau de remplissage maximal prédéfini.

10. Procédé de détection d'au moins une présence de mousse (9₂, 9₃) d'un milieu (9), comprenant :
- la mise à disposition d'une installation de bioréacteur (50) pourvue d'au moins un récipient à usage unique (1) pour la réception du milieu (9), qui peut comprendre la mousse (9₂, 9₃), et d'un orifice d'air d'échappement (12b) ;
- la disposition en au moins deux emplacements de disposition (A1-A3 ; B1-B6 ; C1, C2) de l'installation de bioréacteur (50) d'au moins deux unités de capteur capacitif (10_{A}, 10_{B},10_{C}), les unités de capteur capacitif présentant respectivement au moins un système d'électrodes (19) pour une mesure capacitive et une unité de capteur (10_{A}, 10_{B},10_{C}) des au moins deux unités de capteur capacitif (10_{A}, 10_{B},10_{C}) étant disposée :
à un premier emplacement de disposition (B2) dans le voisinage immédiat de l'orifice d'air d'échappement (12b) ou
à un deuxième emplacement de disposition (C1) au ou dans le voisinage spatial d'un tuyau d'air d'échappement (12a) de l'installation de bioréacteur (50), le tuyau d'air d'échappement (12a) étant en liaison fluidique avec l'orifice d'air d'échappement (12b),
- la détection de données concernant la présence de mousse en les au moins deux emplacements de mesure sur la base des mesures capacitives ; et
- la transmission des données captées concernant la présence de mousse (9₂, 9₃) à au moins une unité de contrôle (14) pour contrôler, commander et/ou réguler une formation de mousse (9) dans l'installation de bioréacteur (50) sur leur base au moyen d'unités de capteur capacitif (10_{A}, 10_{B},10_{C}),
- le déclenchement d'une mesure telle qu'un arrêt d'un agitateur et/ou d'un gazage lors du déclenchement de la deuxième unité de capteur (10_{A}, 10_{B},10_{C}).

11. Procédé selon la revendication 10, comprenant la surveillance, la commande et/ou la régulation de la formation de mousse (9) dans l'installation de bioréacteur (50) au moyen de l'unité de contrôle (14) sur la base des données transmises.

12. Procédé selon la revendication 10 ou 11, dans lequel la commande et/ou la régulation comprend une amenée d'une substance (23), qui est conçue pour empêcher une formation de mousse ou du moins pour la réduire, lorsque la présence de mousse est détectée au moyen d'au moins une unité de capteur capacitif, de préférence lorsque la présence de mousse est détectée au moyen d'au moins une unité de capteur capacitif à un emplacement de disposition en dehors du récipient à usage unique, en particulier au moyen d'une unité de capteur en arc capacitif.

13. Procédé selon l'une des revendications 10 à 12, comprenant :
- le déclenchement d'une alarme lorsque la présence de mousse est détectée au moyen d'au moins une unité de capteur capacitif (10_{A}, 10_{B},10_{C}) à un emplacement de disposition (A1-A3) dans l'espace intérieur (2) du récipient à usage unique (1) et/ou à un emplacement de disposition (B1-B6) en dehors du récipient à usage unique (1) ; et/ou
- le déclenchement d'un arrêt d'urgence lorsque la présence de mousse est détectée au moyen d'au moins un capteur (10_{A}, 10_{B},10_{C}) à un emplacement de disposition (B1-B6) en dehors du récipient à usage unique (1).

14. Procédé de montage d'un système (100) selon l'une des revendications 1 à 9 pour la détection d'au moins une présence de mousse (9₂, 9₃) d'un milieu (9) dans une installation de bioréacteur (50), comprenant :
- la mise à disposition d'une installation de bioréacteur (50) pourvue d'au moins un récipient à usage unique (1) pour la réception du milieu (9), qui peut comprendre la mousse (9₂, 9₃) ;
- la mise à disposition d'au moins deux unités de capteur capacitif (10_{A}, 10_{B},10_{C}), les unités de capteur capacitif présentant respectivement au moins un système d'électrodes (19) pour une mesure capacitive ;
- la disposition des au moins deux unités de capteur capacitif (10_{A}, 10_{B},10_{C}) au niveau respectivement d'un emplacement de disposition (A1-A3 ; B1-B6 ; C1, C2) de l'installation de bioréacteur (50) ; et
- la connexion des au moins deux unités de capteur capacitif (10_{A}, 10_{B},10_{C}) au niveau d'au moins une unité de contrôle (14) pour contrôler, commander et/ou réguler une formation de mousse (9) dans l'installation de bioréacteur (50) sur la base des mesures capacitives.
